# EUROPEAN PATENT APPLICATION

(11) **EP 1 757 692 A1**
(43) Date of publication of application: **28.02.2007**
(21) Application number: 05734386.5
(22) Date of filing: 20.04.2005
(51) Int. Cl.: C12N 15/12, A61K 45/00, A61P 11/00, A61P 35/00, C07K 14/82, C07K 16/32, C12N 5/10, C12P 21/02, C12Q 1/68, G01N 33/15, G01N 33/50, G01N 33/53, G01N 33/566

(54) **GENE ENCODING GUANINE NUCLEOTIDE EXCHANGE FACTOR BINDING TO RhoA**

(30) Priority: 21.04.2004 JP 2004125997
(71) Applicant: DAIICHI PHARMACEUTICAL CO., LTD., Chuo-ku, Tokyo 103-8234 (JP); Kazusa DNA Research Institute Foundation, Kisarazu-shi, Chiba 292-0818 (JP)
(72) Inventor: OHARA, Osamu, KAZUSA DNA RESEARCH INST. FOUNDATION, Kisarazu-shi, Chiba 2920818 (JP); NAGASE, Takahiro, KAZUSA DNA RESEARCH INST. FOUND., Kisarazu-shi, Chiba 2920818 (JP); OHISHI, Michio, KAZUSA DNA RESEARCH INST. FOUND., Kisarazu-shi, Chiba 2920818 (JP); YOKOTA, Hiroshi, Daiichi Pharmaceutical Co., Ltd., Edogawa-ku, Tokyo 1348630 (JP); KAMIDA, Osamu, Daiichi Pharmaceutical Co., Ltd., Edogawa-ku, Tokyo 1348630 (JP)
(74) Representative: De Clercq, Ann G. Y.
(86) International application number: PCT/JP2005/007526
(87) International publication number: WO 2005/103257

(57) **Abstract**

A gene encoding a novel protein that binds to a Rho family protein being one group of small GTP binding proteins and is capable of exhibiting a GEF activity, namely, a polynucleotide shown by the nucleotide sequence set forth in SEQ ID NO: 1, or SEQ ID NO: 3, or the complementary strand, the equivalents of the polynucleotide, a protein encoded by the polynucleotide, a vector containing the polynucleotide, a transformant containing the vector, an antibody against the protein encoded by the polynucleotide, a method of identifying a compound that inhibits the function of the protein encoded by the polynucleotide and/or the expression of the polynucleotide, a method of determining a disease, a pharmaceutical composition, and a reagent kit are provided.

## Description

### TECHNICAL FIELD

The present invention relates to proteins capable of binding to Rho family proteins that are one group of small GTP binding protein and capable of exhibiting an activity of a guanine nucleotide exchange factor (GEF) for the Rho family proteins, and to a polynucleotide encoding the protein. Specifically, the present invention relates to a protein that binds to Rho family small GTP binding protein RhoA, a polynucleotide encoding the protein, a recombinant vector containing the polynucleotide, and a transformant being transfected with the recombinant vector. Further, the present invention relates to a method of producing the protein, and an antibody raised against the protein. Furthermore, the present invention relates to a method of identifying a compound that inhibits the function of the protein and/or the expression of the polynucleotide. Further, the present invention relates to a method of diagnosing tonsil tumors and/or bronchial tumors, comprising measuring the amount of expression of the polynucleotide. Furthermore, the present invention relates to an agent for preventing and/or treating tonsil tumors and/or bronchial tumors containing an inhibitor for the function of the protein and/or an inhibitor for the expression of the polynucleotide, and a method of preventing and/or treating tonsil tumors and/or bronchial tumors, comprising using an inhibitor for the function of the protein and/or an inhibitor for the expression of the polynucleotide. Further, the present invention relates to a reagent kit containing at least one of the following components: the protein, the polynucleotide, the recombinant vector, the transformant and the antibody.

### BACKGROUND OF INVENTION

A Rho family small GTP binding protein (hereinafter, may be simply referred to as a Rho family protein) belongs to one group of small GTP binding proteins (hereinafter, may be simply referred to as a small G protein). A small G protein works as a signal amplifier between a cell membrane receptor, and an effector participating in an intracellular signal transduction pathway. Further, the small G protein specifically binds to guanosine 5'-triphosphate (GTP) or guanosine 5'-diphosphate (GDP), and shows an enzyme activity of hydrolyzing the bound GTP to GDP. When an extracellular signaling substance binds to a cell membrane receptor, its signal is transduced to a small G protein, which subsequently leads to a reaction exchanging a GDP bound to the small G protein for an intracellular GTP (hereinafter, the reaction may be abbreviated to GDP/GTP exchange reaction). Consequently, an active small G protein that is a GTP binding form is generated. The active small G protein acts on the effector to amplify the signal. Then, the GTP binding form of small G protein hydrolyzes the bound GTP to GDP by its enzyme activity and thereby become inactivated. Thus, the small G protein works as a molecular switch in an intracellular signal transduction pathway by exchanging the guanine nucleotides.

Cdc42, Rac1, RhoA and the like, are known as Rho family proteins. Cdc42 regulates firopodia formation in a fibroblast. Rac1 regulates superoxide production in leukocytes and macrophages, while it regulates cell membrane ruffling and lamellipodia formation in fibroblasts. Further, Cdc42 and Rac1 are capable of activating the c-Jun N-terminal kinase signal transduction pathway. Thus, Rho family proteins are involved in various kinds of cell function by regulating the intracellular signal transduction. For example, cytoskeleton restructuring, cell adhesion, gene expression, and the like, are known as a Rho family protein-mediated cell function. Such functions mediated by Rho family proteins are considered to regulate morphogenesis during ontogeny, migration of leukocyte and the like, axon degeneration, tumor metastasis, and invasion.

Rho guanine nucleotide exchange factor (hereinafter, may be abbreviated as Rho-GEF) is a member of a family of proteins involved in the molecular switching of a Rho family protein. Rho-GEF can function to accelerate the GDP/GTP exchange reaction of a Rho family protein, and can thereby convert the Rho family protein from a GDP-bound inactive form to a GTP-bound active form. Rho-GEF plays an important role through this function, in regulating Rho family protein-mediated intracellular signal transduction. Hereinafter, to accelerate the GDP/GTP exchange reaction and thereby to convert the Rho family protein from a GDP-bound inactive form to a GTP-bound active form may be referred to as a GEF activity or Rho family protein activation.

Rho-GEF has a characteristic domain structure, such as a Db1 homology domain (hereinafter, may be abbreviated as DH domain) and a pleckstrin homology domain (hereinafter, may be abbreviated as PH domain). The DH/PH tandem structure is a typical domain structure for Rho-GEF. Hereinafter, the tandem structure ofDH domain and PH domain may be referred to as DH/PH domain.

The DH/PH domain is an important domain participating in the Rho-GEF-mediated activation of a Rho family protein, and is considered to be an active domain of Rho-GEF. For example, it has been reported that a protein, which comprises a C-terminal region of the amino acid sequence of proto-Db1, and contains a DH/PH domain, activated a Rho family protein (Non-Patent Reference 1). proto-Db1 is a prototype of Rho-GEF. Specifically, this report showed that a protein consisting of a C-terminal region within the entire amino acid sequence of proto-Db1 with 925 amino acids, which was generated by deletion of the N-terminal amino acid residues from the 1^{st} to the 497^{th}, activated a Rho family protein, and consequently participated in cellular transformation. From these facts, the activation of proto-Db1 is considered to be an oncogenic activation. Hereinafter, a protein that consists of the C-terminal region of proto-Db1 is referred to as an oncogenic-Db1. It has been reported that oncogenic-Db1 bind to RhoA, Cdc42 and Rac1, and that they have a GEF activity for Cdc42 and RhoA while they do not exert a GEF activity to Rac1 (Non-Patent Reference 2).

Vav (Non-Patent Reference 3 and 4), ost (Non-Patent Reference 5), lbc (Non-Patent Reference 6), and the like, are known to be genes that encode proto-Db1 family proteins. These genes are known to be involved in cancer. Further, trio (Non-Patent Reference 7), kalirin (Non-Patent Reference 8), and the like, are reported to be genes that encode proteins working as Rho-GEF. A trio knocked-out mouse shows an abnormal skeletal muscle structure and an abnormal brain structure in embryogenesis. Kalirin is involved in axon formation in neurons. Thus, each protein working as Rho-GEF is involved in a cellular function particular to each kind of protein, and activates a different Rho family protein.

Literatures referred in the present description are listed hereunder.
Non-patent Reference 1: Bi, F. et al., Molecular and Cellular Biology, 2001, Vol. 21, p. 1463-1474
Non-patent Reference 2: Hart, M. J. et al., Journal of Biological Chemistry, 1994, Vol. 269, p. 62-65
Non-patent Reference 3: Katzav, S. et al., EMBO Journal, 1989, Vol. 8, p. 2283-2290
Non-patent Reference4: Costello, P. S. et al., Proceedings of The National Academy of Sciences of The United States of America, 1999, Vol. 96, p. 3035-3040
Non-patent Reference5: Horii, Y. et al., EMBO Journal, 1994, Vol. 13, p. 4776-4786
Non-patent Reference6: Toksoz, D. et al., Oncogene, 1994, Vol. 9, p. 621-628
Non-patent Reference7: O'Brien, S. P. et al., Proceedings of The National Academy of Sciences of The United States of America, 2000, Vol. 97, p. 12074-12078
Non-patent Reference8: Penzes, P. et al., Journal of Neuroscience, 2001, Vol. 21, p. 8426-8434

### DISCLOSURE OF THE INVENTION

### [PROBLEM TO BE SOLVED BY THE INVENTION]

An object of the present invention is to provide a novel Rho-GEF, and a gene encoding the Rho-GEF. Further, the object of the present invention includes providing a recombinant vector that contains the gene, and a transformant transfected with the recombinant vector. Furthermore, an object of the present invention includes providing a method of producing the Rho-GEF, and an antibody recognizing the Rho-GEF. Further, an object of the present invention includes providing a method of identifying a compound that inhibits the function of the Rho-GEF and/or the expression of the gene. Furthermore, an object of the present invention includes providing a method of preventing and/or treating a disease due to the abnormal function of the Rho-GEF and/or the abnormal expression of the gene, a method of diagnosing the disease, and a reagent kit.

### [MEANS FOR SOLVING THE OBJECT]

The present inventors have concentrated their efforts to meet the aforementioned object. Then, the present inventors have discovered a novel gene having a DH/PH domain-coding region characteristic for Rho GEF, and have successfully obtained the gene. Further, the present inventors revealed experimentally that a protein encoded by the gene was bound to a Rho family protein, RhoA. Moreover, the present inventors found that the tissue expression of the gene in a case of tonsil squamous cell carcinoma and a case of bronchial adenoid cystic carcinoma is at least 2.5 times or more higher, and at least 1.5 times or more higher, respectively, compared to that in a respective normal tissue. The present invention has been thus achieved.

In various embodiments, the present invention relates to a polynucleotide shown by the nucleotide sequence set forth in SEQ ID NO: 1 or by the complementary nucleotide sequence, or a polynucleotide encoding a protein shown by the amino acid sequence set forth in SEQ ID NO: 2 or a polynucleotide shown by the complementary nucleotide sequence of the polynucleotide.

The present invention also relates to a polynucleotide selected from the following group, wherein the polynucleotide encodes a protein that binds to RhoA:
(i) a polynucleotide shown by a nucleotide sequence having a homology of at least 70% with the nucleotide sequence of the aforementioned polynucleotide,
(ii) a polynucleotide with a mutation or an induced mutation, such as deletion, substitution, addition of one or several nucleotides in the nucleotide sequence of the aforementioned polynucleotide, and
(iii) a polynucleotide that hybridizes to the aforementioned polynucleotide under stringent conditions.

The present invention further relates to a polynucleotide selected from the following group, wherein the polynucleotide encodes a protein having a guanine nucleotide exchange factor (GEF) activity for RhoA:
(i) a polynucleotide shown by a nucleotide sequence having a homology of at least 70% with the nucleotide sequence of the aforementioned polynucleotide,
(ii) a polynucleotide with a mutation or an induced mutation, such as deletion, substitution, addition of one or more nucleotides in the nucleotide sequence of the aforementioned polynucleotide, and
(iii) a polynucleotide that hybridizes to the aforementioned polynucleotide under stringent conditions.

The present invention still further relates to recombinant vectors containing any one of the aforementioned polynucleotides.

The present invention also relates to transformants that have been transfected with any of the aforementioned recombinant vectors.

The present invention further relates to transformants that have been transfected with any of the aforementioned recombinant vectors and recombinant vectors containing a polynucleotide encoding RhoA.

The present invention still further relates to a protein shown by the amino acid sequence set forth in SEQ ID NO: 2 in the sequence listing.

The present invention also relates to proteins encoded by any one of the aforementioned polynucleotides.

The present invention further relates to methods of producing the aforementioned proteins, comprising a step of culturing the aforementioned transformants.

The present invention still further relates to antibodies that recognize the aforementioned proteins.

The present invention also relates to a method of identifying a compound that inhibits the function of the aforementioned proteins, and/or the expression of any one of the aforementioned polynucleotides. The method can comprise detecting the presence, absence or change in the function and/or the expression of any of the aforementioned proteins or polynucleotides, under conditions where the interaction of a compound with the protein and/or the polynucleotide are allowed, and determining whether the compound inhibits the function of the protein and/or the expression of the polynucleotide.

The present invention further relates to the aforementioned identification method, wherein the function of the protein is a GEF activity for RhoA.

The present invention still further relates to a method of determining whether a tissue specimen derived from a human tonsil tissue is a tissue derived from a human tonsil tumor or not, comprising measuring an amount of expression of any one of the aforementioned polynucleotides in the tissue specimen.

The present invention also relates to the aforementioned determination method, wherein the method determines that the tissue specimen is a tissue derived from a human tonsil tumor where the amount of expression of any one of the aforementioned polynucleotides in the tissue specimen is at least 2.5 times or more higher than that in a control tissue derived from human normal tonsil.

The present invention further relates to a method of determining whether a tissue specimen derived from a human bronchial tissue is a tissue derived from a human bronchial tumor or not, comprising measuring an amount of expression of any one of the aforementioned polynucleotides in the tissue specimen.

The present invention still further relates to the aforementioned determination method, wherein the method determines that the tissue specimen is a tissue derived from a human bronchial tumor where the amount of expression of any one of the aforementioned polynucleotides in the tissue specimen is at least 1.5 times or more higher than that in a control tissue derived from human normal bronchia.

The present invention also relates to agents for preventing and/or treating tonsil tumors and/or bronchial tumors, comprising a compound that inhibits the function of the aforementioned proteins, and/or a compound that inhibits the expression of any one of the aforementioned polynucleotides, as an active ingredient.

The present invention further relates to methods of preventing and/or treating tonsil tumors and/or bronchial tumors, comprising using a compound that inhibits the function of the aforementioned proteins, and/or a compound that inhibits the expression of any one of the aforementioned polynucleotides.

The present invention still further relates to a reagent kit containing at least one of the aforementioned proteins, the aforementioned polynucleotides, the aforementioned recombinant vectors, the aforementioned transformants, and the aforementioned antibodies.

### ADVANTAGE OF THE INVENTION

The present invention can provide polynucleotides encoding novel proteins that bind to Rho family proteins, and proteins encoded by the polynucleotides. A present protein was bound to a Rho family protein, RhoA. The proteins have a DH/PH domain that is considered to be an active domain of Rho-GEF in the amino acid sequence. Thus, the present proteins can be considered to bind to Rho proteins, such as RhoA, and to exhibit a GEF activity.

The present invention can also provide recombinant vectors containing the polynucleotides, and transformants transfected with the recombinant vectors. Further, the present invention can provide methods of producing the proteins, and antibodies specific to the proteins. Furthermore, the present invention can provide methods of identifying a compound that inhibits the function of the proteins and/or the expression of the polynucleotides. Further, the present invention can provide methods of diagnosing a tumor disease such as tonsil tumors and bronchial tumors, comprising measuring an amount of expression of the polynucleotides. Furthermore, the present invention can provide agents for preventing and/or treating a tumor disease such as tonsil tumors and bronchial tumors, comprising an inhibitor of the function of the proteins and/or an inhibitor of the expression of the polynucleotides, as an active ingredient. Further, the present invention can provide methods of preventing and/or treating a tumor disease such as tonsil tumors and bronchial tumors, comprising using the inhibitor of the function of the proteins and/or the inhibitor of the expression of the polynucleotides. Furthermore, the present invention can provide a reagent kit comprising at least one of the following components: the protein; the polynucleotide; the recombinant vector; the transformant; and the antibody.

The present invention allows elucidation and regulation of the signal transduction pathway and cellular function which are mediated by Rho family proteins such as RhoA. Further, the present invention allows diagnosis, prevention and/or treatment of a disease due to an abnormal function of the present proteins and/or an abnormal expression of the present polynucleotides, for example, a tumor disease, more specifically, tonsil tumors and bronchial tumors.

### BRIEF DESCRIPTION OF THE DRAWINGS

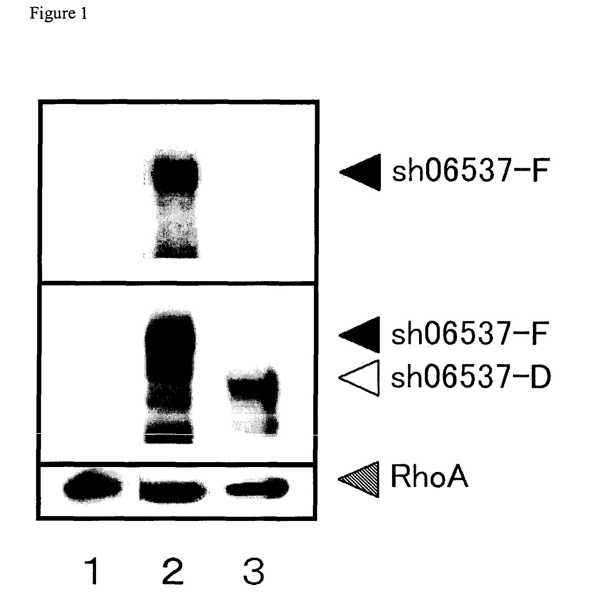

Figure 1 shows a band corresponding to binding of a protein encoded by sh06537-F to RhoA, that was detected (lane 2 in the upper panel) in a cell lysate prepared from a cell into which a sh06537-F expression vector was co-transfected with a RhoA expression vector. The sh06537-F expression vector allows the expression of a protein encoded by a polynucleotide shown by the nucleotide sequence set forth in SEQ ID NO: 1 in the sequence listing, as a C-terminal 3x FLAG-tagged protein. A pull down assay was used for measuring the binding. On the other hand, such a band was not detected (lane 3 in the upper panel) in a cell lysate prepared from a cell into which a vector constructed by using DNA, that consists of a partial sequence of sh06537 lacking a DH/PH domain coding region (sh06537-D), was co-transfected with a RhoA expression vector. Further, such a band was not detected (lane 1 in the upper panel) in a cell lysate prepared from a cell into which only a RhoA expression vector was transfected. The middle panel shows results of detecting a protein encoded by sh06537-F, or a protein encoded by sh06537-D, in each cell lysate sample, by SDS-PAGE using an anti-FLAG antibody. The lower panel shows results of detecting a GST-fusion RhoA in each cell lysate sample, by SDS-PAGE using an anti-GST antibody. The amount of the protein encoded by sh06537-F, or the protein encoded by sh06537-D, was almost the same in each cell lysate sample (lanes 2 and 3 in the middle panel). Further, the amount of RhoA was almost the same in each cell lysate sample (lanes 1 to 3 in the lower panel). (Example 3)

### DETAILED DESCRIPTION OF THE INVENTION

Embodiments of the present invention are explained in further detail below.

In the present invention, the term "polynucleotide" may be used as a generic term which includes the following: an isolated full-length DNA and/or RNA; a synthetic full-length DNA and/or RNA; an isolated DNA oligonucleotide and/or RNA oligonucleotide; or a synthetic DNA oligonucleotide and/or RNA oligonucleotide. Such DNA and/or RNA used herein comprise two or more nucleotides.

In the present invention, the term "protein" may be used as a generic term which includes the following: an isolated or a synthetic full-length protein; an isolated or a synthetic full-length polypeptide; and an isolated or a synthetic full-length oligopeptide. A protein, a polypeptide or an oligopeptide used herein comprises two or more amino acids. Hereinafter, an amino acid may be represented by a single letter or by three letters.

### (polynucleotide)

An aspect of the present invention relates to a novel polynucleotide. The present polynucleotide was identified and isolated from a cDNA library derived from human spleen tissue as a gene having a region encoding a DH/PH domain that is a characteristic domain for Rho-GEF.

The polynucleotide according to the present invention can be a polynucleotide shown by the nucleotide sequence set forth in SEQ ID NO: 1 in the sequence listing, or by the complementary nucleotide sequence thereof. The polynucleotide shown by the nucleotide sequence set forth in SEQ ID NO: 1 is 5541 bp long and contains an open reading flame (ORF) encoding 1597 amino acid residues (SEQ ID NO: 2). The region in the nucleotide sequence set forth in SEQ ID NO: 1, which consists of the nucleotides from the 3706^{th} to the 4245^{th}, encodes a DH domain consisting of 180 amino acid residues from the 1178^{th} valine (Val) to the 1357^{th} asparagine (Asn) of the amino acid sequence set forth in SEQ ID NO: 2. The region in the nucleotide sequence set forth in SEQ ID NO: 1, which consists of the nucleotides from the 4345^{th} to the 4680^{th}, encodes a PH domain consisting of 112 amino acid residues from the 1391^{st} leucine (Leu) to the 1502^{nd} glutamic acid (Glu) of the amino acid sequence set forth in SEQ ID NO: 2. The region in the nucleotide sequence set forth in SEQ ID NO: 1, which consists of the nucleotides from the 3706^{th} to the 4680^{th}, encodes a DH/PH domain consisting of 325 amino acid residues from the 1178^{th} valine (Val) to the 1502^{nd} glutamic acid (Glu) of the amino acid sequence set forth in SEQ ID NO: 2.

A polynucleotide encoding a protein shown by the amino acid sequence set forth in SEQ ID NO: 2, or a polynucleotide shown by the complementary nucleotide sequence of the polynucleotide is also included in the scope of the present invention.

The polynucleotide according to the present invention can be preferably a polynucleotide encoding a protein that binds to Rho family proteins, or a polynucleotide shown by the complementary nucleotide sequence of the polynucleotide. The phrase "binding between proteins" used in the present invention refers to the interaction of a certain protein (protein A) with an another certain protein (protein B) so as to form a complex by non-covalent bond such as hydrogen bond, hydrophobic bond, electrically static interaction or the like. The binding of protein A to protein B only at the portion of these molecules is enough to be referred as "the binding" mentioned herein. For example, an amino acid which is not involved in the binding of protein A to protein B may be contained in the amino acid that constitutes protein A or protein B.

The polynucleotide shown by the nucleotide sequence set forth in SEQ ID NO: 1 encodes a protein that was bound to a Rho family protein, RhoA. Concretely, in an animal cell into which the polynucleotide shown by the nucleotide sequence set forth in SEQ ID NO: 1 was co-expressed with a gene encoding RhoA, the binding of a protein encoded by the polynucleotide to RhoA was detected (See Example 3). On the other hand, in an animal cell into which the polynucleotide shown by the nucleotide sequence set forth in SEQ ID NO: 3 was co-expressed with a gene encoding RhoA, no binding was detected between a protein encoded by the polynucleotide and RhoA (See Example 3). The polynucleotide shown by the nucleotide sequence set forth in SEQ ID NO: 3 is a polynucleotide shown by the nucleotides from the 175^{th} nucleotide to the 3693^{rd} nucleotide of the nucleotide sequence set forth in SEQ ID NO: 1. The polynucleotide shown by the nucleotide sequence set forth in SEQ ID NO: 3 lacks a DH/PH domain coding region that is present in the polynucleotide shown by the nucleotide sequence set forth in SEQ ID NO: 1. Thus, the polynucleotide (SEQ ID NO: 3) lacking a DH/PH domain coding region encodes a protein that did not bind to RhoA. These findings revealed that a DH/PH domain is participating in the binding of a protein encoded by the polynucleotide shown by the nucleotide sequence set forth in SEQ ID NO: 1, to RhoA. A DH/PH domain is known to be important for the binding of Rho-GEF to a Rho family protein, and for a GEF activity of Rho-GEF.

The polynucleotides according to the present invention encode proteins that have a DH/PH domain in the structure, and bind to Rho family proteins. A DH/PH domain is important for the binding of Rho-GEF to a Rho family protein, and for a GEF activity of Rho-GEF. Therefore, the inventors believe that the polynucleotides according to the present invention encode proteins that bind to Rho family proteins and exhibit a GEF activity for the Rho family proteins. In other words, the present polynucleotides encode proteins that bind to Rho family proteins and activate the Rho family proteins.

The phrase "GEF activity for Rho family proteins" or "activation of Rho family proteins" means to exchange guanosine 5'-diphosphate (GDP), that is bound to a Rho family protein, for guanosine 5'-triphosphate (GTP), and thereby to convert the Rho family protein from a GDP-bound inactive form to a GTP-bound active form. This exchange reaction comprises a dissociation reaction of GDP from a Rho family protein, and a binding reaction of GTP to the resultant Rho family protein, without a nucleotide being bound. Specifically, the phrase "GEF activity for RhoA" or "activation of RhoA" means to convert RhoA from an inactive form to an active form via an exchange reaction of RhoA-bound GDP for intracellular GTP.

In the present specification, the phrase "GEF activity for Rho family proteins" and the phrase "activation of Rho family proteins" can be used interchangeable.

A Rho family protein, which is activated by the proteins encoded by the polynucleotides according to the present invention, may be preferably exemplified by RhoA. In the present invention, the phrase "Rho family protein" may preferably direct to RhoA. A Rho family protein is not limited to this specific example and can be any Rho family protein, as long as it is activated by the proteins encoded by the present polynucleotides. It can be preferably exemplified by Rho family proteins that are activated by being bound with the proteins encoded by the present polynucleotides. The binding of the proteins encoded by the present polynucleotides to Rho family proteins can be measured by using, for example, a pull-down assay (see Example 3). In addition, the activation of Rho family proteins by the proteins encoded by the present polynucleotides can be measured by using, for example, an effector pull-down assay, which is described later.

The amino acid sequence of RhoA and the nucleotide sequence of RhoA gene are set forth in SEQ ID NO: 9 and SEQ ID NO: 8, respectively. RhoA and its gene are not limited to the specific examples shown by the aforementioned sequences. They can be a protein or a gene with a mutation of one or several sites in the aforementioned sequence, as long as it has a function of RhoA generally known. Further, a mutant can be prepared for use by introducing a mutation into one or several sites of the aforementioned sequence in order to enhance or diminish the function of the protein and the gene. RhoA can be produced, for example, by culturing a transformant that was prepared by transfecting with a recombinant vector containing the gene by using well-known gene manipulation techniques.

Polynucleotides according to the present invention can be prepared based on the sequence information concerning the specific example provided by the present invention, such as the polynucleotide shown by the nucleotide sequence set forth in SEQ ID NO: 1 in the sequence listing. The production of the polynucleotide can be carried out easily by using gene manipulation techniques that are well known per se (Sambrook et al., Eds., "Molecular Cloning, A Laboratory Manual, 2nd Edition", 1989, Cold Spring Harbor Laboratory: Muramatsu Masami., Ed., "Labomanual Genetic Engineering", 1988, Maruzen Co., Ltd.).

Specifically, the polynucleotide of the present invention can be acquired by preparing a cDNA library in accordance with an ordinary method, from a suitable source in which expression of the polynucleotide was found, and then selecting a desired clone from the cDNA library. As a cDNA source, various kinds of cells and tissues in which expression of the present polynucleotide was found, or cultured cells derived from these cells and tissues, for example, cells derived from human spleen tissue, or the like, can be used. Isolation of total RNA from these sources, isolation and purification of mRNA, acquisition of cDNA, the cloning thereof, and the like, can each be performed in accordance with an ordinary method. It is also possible to use a cDNA library that was constructed from commercially available polyA⁺ RNA derived from human spleen tissue. A method for selecting a desired clone from a cDNA library is not particularly limited, and any methods generally used can be employed. For example, selection of a desired clone can be performed by using a probe or primer capable of selectively hybridizing to the present polynucleotide. Specifically, a plaque hybridization method, colony hybridization method, or the like, which uses a probe capable of selectively hybridizing to the present polynucleotide, or a combination of these methods, can be employed. As a probe, a polynucleotide chemically synthesized based on the sequence information of the present polynucleotide, and the like, can generally be used. The present polynucleotide or a polynucleotide shown by the partial nucleotide sequence of the present polynucleotide, is suitable for use as a probe, as well. Furthermore, a sense primer and an anti-sense primer, which were designed based on the sequence information of the present polynucleotide, can also be used as such a probe.

Selection of a desired clone from a cDNA library can be performed, for example, by detecting the expression of the protein in each clone, utilizing a known protein expression system, and further determining a biological function of the protein as an indicator. Any known expression system can be used as a protein expression system. For example, a cell free protein expression system can be conveniently used for determining the expression of protein (Madin, K. et al., Proceedings of The National Academy of Sciences of The United States of America, 2000, Vol. 97, p.559-564).

A function of the proteins encoded by the polynucleotides according to the present invention, or a function of the proteins according to the present invention can be, for example, preferably a GEF activity for Rho family proteins, and more preferably a function of binding to Rho family proteins to exhibit a GEF activity. In other words, it can be, for example, preferably a function of activating Rho family proteins, and more preferably a function of binding to Rho family proteins to activate the Rho family proteins.

The polynucleotide according to the present invention can be also prepared preferably by using a DNA/RNA amplification method. For example, a polymerase chain reaction can be used (hereinafter, may be abbreviated as PCR: Ulmer, K.M. Science, 1983, Vol. 219, p.666-671; Ehrlich, H. A., Ed., PCR Technology. Principles and Applications for DNA Amplification, 1989, Stockton Press; and Saiki, R.K. et al., Science, 1985, Vol. 230, p.1350-1354). When the full-length cDNA is difficult to obtain from a cDNA library, a RACE method (Jikken Igaku (Experimental Medicine), 1994, Vol. 12, No. 6, p. 615-618), particularly the 5'-RACE method (Frohman, M. A. et al., Proceedings of The National Academy of Sciences of The United States of America, 1988, Vol. 85, No. 23, p.8998-9002), or the like, can be suitably employed. Primers to be used for PCR can be suitably designed based on the nucleotide sequence information of the polynucleotide, and can be obtained by synthesis in accordance with any conventional method. Isolation and purification of amplified DNA/RNA fragments can be carried out according to any conventional method, such as gel electrophoresis, or the like.

A determination of the nucleotide sequence of DNA thus obtained can be carried out by any conventional method, such as the dideoxy method (Sanger, F. et al., Proceedings of The National Academy of Sciences of The United States of America, 1977, Vol. 74, p.5463-5467), and the Maxam-Gilbert method (Maxam, A.M. et al., Methods in Enzymology, 1980, Vol. 65, p.499-560), or by simply using a commercially available sequencing kit, or the like.

The polynucleotides according to the present invention are not limited to the aforementioned polynucleotides. They can include polynucleotides having a sequence homology with the aforementioned polynucleotides, which preferably encode proteins that bind to Rho family proteins, and more preferably encode proteins that bind to Rho family proteins to exhibit a GEF activity, or polynucleotides shown by the complementary nucleotide sequences of the polynucleotides. A suitable sequence homology with the entire base sequence is normally 50% or more, preferably at least 70%. It is more preferably 70% or more, even more preferably 80% or more, and still more preferably 90% or more. Further, polynucleotides having a DH/PH domain coding region are still more preferable for such polynucleotides. A suitable sequence homology with the DH/PH domain coding region is preferably at least 70%. It is more preferably 70% or more, even more preferably 80% or more, and still more preferably 90% or more. In addition, it is still more preferable that the DH/PH domain has its specific function such as a function of binding to Rho family proteins to exhibit a GEF activity.

The polynucleotides according to the present invention include polynucleotides shown by a nucleotide sequence with a mutation, such as deletion, substitution, addition or insertion, of one or more nucleotides in the nucleotide sequences of the aforementioned DNA, or polynucleotides shown by complementary nucleotide sequences. The number of mutated nucleotides is, for example, from 1 to 100, preferably from 1 to 30, more preferably from 1 to 20, even more preferably from 1 to 10, and still more preferably from 1 to several in number. The extent of mutation, the position of a mutation, and the like, are not particularly limited, as long as the polynucleotides with mutations preferably encode proteins that bind to Rho family proteins, and more preferably encode proteins that bind to Rho family proteins to exhibit a GEF activity. A polynucleotide that encodes a protein having a DH/PH domain is still more preferable. The polynucleotides with mutations may be natural polynucleotides, or may be mutated polynucleotides. Further, they also may be polynucleotides prepared by introducing a mutation into a natural gene. Techniques for introducing a mutation are known in the art. For example, site-directed mutagenesis, genetic homologous recombination, primer extension, PCR, and the like, can be used independently or in suitable combinations. Specifically, for example, a method described in publications (Sambrook et al., Eds., "Molecular Cloning, A Laboratory Manual, 2nd Edition", 1989, Cold Spring Harbor Laboratory; and Muramatsu Masami., Ed., "Labomanual Genetic Engineering", 1988, Maruzen Co., Ltd.), or a modified method thereof, can be used for conducting the introduction of a mutation. In addition, Ulmer's techniques (Ulmer, K.M. Science, 1983, Vol. 219, p.666-671) can also be utilized.

The polynucleotides of the present invention can also be polynucleotides that hybridize to the aforementioned polynucleotides under stringent conditions. A hybridization condition can be found, for example, in a method described in publications (Sambrook et al., Eds., "Molecular Cloning, A Laboratory Manual, 2nd Edition", 1989, Cold Spring Harbor Laboratory), or the like. More specifically, the phrase "under stringent conditions" refers to, for example, a condition of heating at 42 °C in a solution containing 6× SSC, 0.5% SDS and 50% formamide, and then washing at 68 °C in a solution containing 0.1× SSC and 0.5% SDS. Such polynucleotides are not required to have complementary sequences with the present polynucleotides, as long as they hybridize to the present polynucleotides. It is desirable that a polynucleotide preferably encodes a protein having a DH/PH domain. It is more desirable that the encoded protein is a protein that binds to Rho family proteins, and more preferable a protein that binds to Rho family proteins to exhibit a GEF activity.

The polynucleotides of the present invention also include oligonucleotides shown by a partial nucleotide sequence of a given region of the aforementioned polynucleotides. The minimum unit of such an oligonucleotide consists of consecutive nucleotides within the region, preferably of 5 or more, more preferable of 10 or more, and even more preferably of 20 or more consecutive nucleotides. Specifically, an oligonucleotide shown by the nucleotide sequence set forth in SEQ ID NO: 5, or SEQ ID NO: 6, can be preferably exemplified. These oligonucleotides can be used, for example, as primers for amplifying the present genes or the present gene fragments, and as probes for detecting the present genes or their transcription products thereof. These oligonucleotides can be prepared by designing a desired sequence based on the nucleotide sequence information of the present polynucleotides, and then synthesizing them using a well-known chemical synthesis method. An automated DNA/RNA synthesizer can be conveniently used for preparing the oligonucleotides.

The polynucleotides of the present invention are preferably human derived polynucleotides. However, the present invention includes polynucleotides derived from mammals, for example, a polynucleotide derive from mouse, horse, sheep, cow, dog, monkey, cat, bear, rat, rabbit or the like, as long as the polynucleotide is a polynucleotide that has a sequence homology with the present polynucleotides, and that encodes a protein that binds to Rho family proteins to exhibit a GEF activity. A suitable sequence homology with the entire base sequence is normally 50% or more, preferably at least 70%. It is more preferably 70% or more, even more preferably 80% or more, and still more preferably 90% or more. Further, polynucleotides having a DH/PH domain coding region are more preferable for such polynucleotides. A suitable sequence homology with the DH/PH domain coding region is preferably at least 70%. It is more preferably 70% or more, even more preferably 80% or more, and still more preferably 90% or more.

The polynucleotides of the present invention also may have a desired gene at the 5'-terminal side or the 3'-terminal side thereof, as long as the expression of the polynucleotides or the function of proteins encoded by the polynucleotides is not inhibited. A gene capable of being added to the present polynucleotide can be specifically exemplified by the genes of enzymes, such as glutathione S-transferase (GST), β-galactosidase, horseradish peroxidase (HRP) or alkaline phosphatase (ALP), or of tag peptides such as His-tag, Myc-tag, HA-tag, FLAG-tag or Xpress-tag. One of these genes, or multiple kinds of these genes in combination, can be added to the present polynucleotides. Addition of these genes can be performed by using conventional gene manipulation techniques, and is useful to facilitate detection of a gene or mRNA.

### (vector)

Another aspect of the present invention relates to recombinant vectors containing a polynucleotide of the present invention. The recombinant vectors can be prepared by inserting the present polynucleotides into a suitable vector DNA.

The vector DNA is not particularly limited, as long as it can be replicated within a host, and can be suitably selected in accordance with the kind of host and purpose of use. The vector DNA may be vector DNA obtained by extracting natural DNA, or may be vector DNA lacking a part of DNA other than a segment necessary for replication. Typical vector DNAs include, for example, a vector DNA derived from a plasmid, a bacteriophage or a virus. A plasmid DNA can be exemplified by a plasmid derived from *Escherichia coli,* a plasmid derived from *Bacillus subtilis,* or a plasmid derived from yeast. A bacteriophage DNA can be exemplified by a λ phage. Vector DNA derived from a virus can be exemplified by a vector derived from an animal virus, such as a retrovirus, vaccinia virus, adenovirus, papovavirus, SV 40, fowlpox virus, and pseudorabies virus, or a vector derived from an insect virus such as baculovirus. Further, vector DNA derived from a transposon, an insertion element, a yeast chromosome element, or the like, may be exemplified. Alternatively, a vector DNA prepared by combining two or more of these, for example, a vector DNA (cosmid, phagemid or the like) prepared by combining genetic elements of a plasmid and a bacteriophage, may be exemplified.

Any vector DNA can be used in accordance with the desired purpose, for example, an expression vector, cloning vector or the like. The recombinant expression vector containing the polynucleotide of the present invention is useful for the production of proteins encoded by the present polynucleotides.

It is necessary for the polynucleotide of the present invention to be incorporated into vector DNA in such a way as to allow the function of the polynucleotide to appear. The vector DNA contains at least one of the present polynucleotides and a promoter, as construction elements. In addition to these elements, as desired, a genetic sequence that encodes information relating to replication and control, may be incorporated in combination into the vector DNA, by using a well-known method. Such a genetic sequence can be exemplified by a ribosome binding sequence, terminator, signal sequence, cis element such as an enhancer, splicing signal, and a selective marker such as dihydrofolate reductase gene, ampicillin-resistant gene and neomycin-resistant gene. The vector DNA may contain one or more kinds of genetic sequences selected from the aforementioned members.

As a method of incorporating the polynucleotide, according to the present invention, into a vector DNA, any known method can be employed. For example, a method may be used which comprises cleaving a gene containing the present polynucleotide at specific sites, by treating it with suitable restriction enzymes, and then mixing it with a similarly treated vector DNA, for ligation using a ligase. Alternatively, a desired recombinant vector may be prepared by using a method that comprises ligating one of the present polynucleotides with a suitable linker, and then inserting it into the multi-cloning site of a vector, suitable for the desired purpose.

### (transformant)

Further aspect of the present invention relates to transformants obtained by transforming a host with the recombinant vectors according to the present invention. A transformant, prepared by introducing a recombinant expression vector that contains a polynucleotide according to the present invention, is useful for producing a protein encoded by the present polynucleotides. The present transformants may further incorporate one or more kinds of vector DNAs, each containing a desired gene other than the present polynucleotides. A vector DNA that contains a desired gene other than a present polynucleotide, can be exemplified by vector DNA that contains a gene encoding a Rho family protein, such as, RhoA, or the like. A transformant prepared by transfecting with both of the expression vectors, one of which contains a present polynucleotide and the other contains a gene encoding a Rho family protein, may be used for a method of identifying a compound that inhibits or enhances a GEF activity of a protein encoded by a present polynucleotide for Rho family proteins. That is to say, the transformant may be used for a method of identifying a compound that inhibits or enhances the activation of Rho family proteins. Such a transformant can be preferably exemplified by a transformant prepared by transfecting with both recombinant vectors, one of which contains the present polynucleotide and the other contains a gene encoding RhoA.

Any suitable prokaryotes and eukaryotes can be employed as a host. Examples of suitable prokaryotes include bacteria belonging to the *Escherichia* genus, such as, *Escherichia coli,* bacteria belonging to the *Bacillus* genus, such as, *Bacillus subtilis,* bacteria belonging to the *Pseudomonas* genus, such as, *Pseudomonas putida,* and bacteria belonging to the *Rhizobium* genus, such as, *Rhizobium meliloti.* Examples of suitable eukaryotes include yeasts, and animal cells such as insect cells and mammalian cells. Yeasts can be exemplified by *Saccharomyces cerevisiae* and *Schizosaccharomyces pombe.* Insect cells can be exemplified by Sf9 cells and Sf2 cells. Mammalian cells can be exemplified by monkey kidney-derived cells, such as COS cells, Vero cells, Chinese hamster ovary cells (CHO cell), mouse L cells, rat GH3 cells, human FL cells, and 293EBNA cells. It is preferable to use mammalian cells, and more preferable to use 293EBNA cells.

Transformation of a host cell with a recombinant vector can be carried out by utilizing known methods. For example, a standard method described in publications (Sambrook et al., Eds., "Molecular Cloning, A Laboratory Manual, 2nd Edition", 1989, Cold Spring Harbor Laboratory) may be utilized. When gene stability is a consideration, it is preferable to use a method that integrates the gene onto a chromosome. Meanwhile, it is convenient to use an autonomous replication system that utilizes an extranuclear gene. Specifically, calcium phosphate transfection, DEAE-dextran mediated transfection, microinjection, cationic lipid-mediated transfection, electroporation, transduction, scrape loading, ballistic introduction, infection, and the like, may be mentioned.

When employing a prokaryote as a host, it is preferable to use a recombinant vector which is capable of autonomous replication within the bacterium, and is also composed of a promoter, a ribosomal binding sequence, the polynucleotide of the present invention, and a transcription termination sequence. It may also contain a gene that regulates the promoter. When employing bacteria as a host, any promoter may be used, as long as it can lead to expression in bacteria, such as *Escherichia coli.* For example, a promoter derived from *Escherichia coli* or a phage can be used, such as a trp promoter, lac promoter, PL promoter or PR promoter. An artificially designed and modified promoter such as a tac promoter may also be used. A method of introducing a recombinant vector into bacteria is not particularly limited, and any methods that introduce DNA into bacteria can be employed. Preferable examples of such a method include using calcium ions, electroporation, or the like.

When employing a mammalian cell as a host, it is preferable to use the recombinant vector which is capable of autonomous replication within the cell, and is also composed of a promoter, RNA splice site, a polynucleotide of the present invention, polyadenylated site and a transcription termination sequence. As desired, it may also contain an origin of replication. A SRα promoter, SV 40 promoter, LTR promoter, CMV promoter, and the like, can be used as a promoter. An early gene promoter of cytomegalovirus, and the like, may be used, as well. As a method of introducing the recombinant vector into a mammalian cell, preferably, for example, electroporation, the calcium phosphate technique, lipofection, or the like, may be used. A most preferable method to be used may be lipofection.

When using yeast as a host, the promoter is not particularly limited, as long as it can lead to expression in yeast. Examples of such a promoter include the gal1 promoter, gal10 promoter, heat shock protein promoter, MFα1 promoter, PHO5 promoter, PGK promoter, GAP promoter, ADH promoter, and AOX1 promoter. A method of introducing a recombinant vector into yeast is not particularly limited, as long as it is a method that introduces the DNA into the yeast. Preferable examples of such a method include electroporation, a spheroplast method, a lithium acetate method or the like.

When using an insect cell as a host, it is preferable to use a calcium phosphate technique, lipofection, or electroporation for a method of introducing a recombinant vector.

### (protein)

A further aspect of the present invention relates to proteins encoded by a polynucleotide according to the present invention.

The protein according to the present invention can be, for example, a protein encoded by the polynucleotide shown by the nucleotide sequence set forth in SEQ ID NO: 1. More specifically, the protein can be a protein shown by the amino acid sequence set forth in SEQ ID NO: 2. The protein shown by the amino acid sequence set forth in SEQ ID NO: 2 has a DH domain composed of the amino acid residues from the 1178^{th} valine (Val) to the 1357^{th} asparagine (Asn). The protein has a PH domain composed of the amino acid residues from the 1391^{st} leucine (Leu) to the 1502^{nd} glutamic acid (Glu). Thus, the protein has a DH/PH domain composed of the amino acid residues from the 1178^{tth} valine (Val) to the 1502^{nd} glutamic acid (Glu).

A protein according to the present invention may be preferably a protein that binds to Rho family proteins. The protein encoded by the polynucleotide shown by the nucleotide sequence set forth in SEQ ID NO: 1, was bound to a Rho family protein, RhoA. Specifically, in an animal cell into which the polynucleotide shown by the nucleotide sequence set forth in SEQ ID NO: 1 was co-expressed with a gene encoding RhoA, the binding of a protein encoded by the polynucleotide to RhoA was detected (See Example 3). The polynucleotide shown by the nucleotide sequence set forth in SEQ ID NO: 1, encodes the protein shown by the amino acid sequence set forth in SEQ ID NO: 2. Therefore, it can be considered that the protein shown by the amino acid sequence set forth in SEQ ID NO: 2, was bound to RhoA. On the other hand, in an animal cell into which the polynucleotide shown by the nucleotide sequence set forth in SEQ ID NO: 3 was co-expressed with a gene encoding RhoA, no binding was detected between a protein encoded by the polynucleotide and RhoA (See Example 3). The polynucleotide shown by the nucleotide sequence set forth in SEQ ID NO: 3 is a polynucleotide shown by the nucleotides from the 175^{th} nucleotide to the 3693^{rd} nucleotide of the nucleotide sequence set forth in SEQ ID NO: 1. The polynucleotide shown by the nucleotide sequence set forth in SEQ ID NO: 3, encodes a protein shown by the amino acid sequence set forth in SEQ ID NO: 4. The protein shown by the amino acid sequence set forth in SEQ ID NO: 4 lacks a region shown by the amino acid resides from the 1174^{th} amino acid to the 1597^{th} amino acid in the amino acid sequence set forth in SEQ ID NO: 2. The region contains a DH/PH domain. Thus, the protein shown by the amino acid sequence set forth in SEQ ID NO: 4 lacks a DH/PH domain. And, the protein shown by the amino acid sequence set forth in SEQ ID NO: 4 lacking a DH/PH domain did not bind to RhoA. These findings revealed that a DH/PH domain participated in the binding of the protein shown by the amino acid sequence set forth in SEQ ID NO: 2 to RhoA.

A protein according to the present invention has a DH/PH domain in the structure and binds to Rho family proteins. A DH/PH domain is important for the binding of Rho-GEF to Rho family proteins, and further for a GEF activity of Rho-GEF. Therefore, the inventors believe that the protein binds to Rho family proteins to exhibit a GEF activity for the Rho family proteins. More specifically, the inventors believe that the protein shown by the amino acid sequence set forth in SEQ ID NO: 2 binds to a Rho family protein, RhoA, and exhibits a GEF activity. In other words, the inventors believe that the present protein binds to RhoA and activates it.

The protein according to the present invention is not limited to a protein as exemplified above, and any protein is included in the scope of the present invention, as long as it is encoded by a polynucleotide according to the present invention. Preferably, a protein that is encoded by a present polynucleotide and that binds to Rho family proteins may be included. More preferably, a protein that is encoded by a present polynucleotide and that binds to Rho family proteins to exhibit a GEF activity may be included. Such a protein can be exemplified by a protein that is encoded by a polynucleotide shown by a nucleotide sequence having a homology of at least 70% with the nucleotide sequence of any one of the polynucleotides selected from the group consisting of the following: a polynucleotide shown by the nucleotide sequence set forth in SEQ ID NO: 1, or its complementary nucleotide sequence; and a polynucleotide encoding the protein shown by the amino acid sequence set forth in SEQ ID NO: 2, or a polynucleotide shown by the complementary nucleotide sequence of the polynucleotide, where the protein has a GEF activity for a Rho family protein. Further, the present protein also includes a protein that is encoded by a polynucleotide shown by a nucleotide sequence with a mutation or a induced mutation, such as deletion, substitution, addition or the like, of one or more nucleotides in the nucleotide sequence of any one of polynucleotides selected from the aforementioned polynucleotide group, where the protein is encoded by a polynucleotide encoding a protein that has a GEF activity for a Rho family protein. Furthermore, the present protein may also be exemplified by a protein encoded by a polynucleotide that hybridizes to any one of the polynucleotides selected from the aforementioned polynucleotide group, under stringent conditions, and encodes a protein that preferably bind to a Rho family protein, more preferably bind to a Rho family protein to show a GEF activity for a Rho family protein.

A protein according to the present invention can be more specifically, for example, a protein that has a sequence homology with the protein shown by the amino acid sequence set forth in SEQ ID NO: 2, and that binds to a Rho family protein. More preferably, it may be a protein that has a sequence homology with the protein shown by the amino acid sequence set forth in SEQ ID NO: 2, and that binds to a Rho family protein to exhibit a GEF activity. A suitable sequence homology with the entire amino acid sequence is normally 50% or more, preferably at least 70%. It is more preferably 70% or more, even more preferably 80% or more, and still more preferably 90% or more. Further, a polypeptide having a DH/PH domain is still more preferable. A suitable sequence homology with the DH/PH domain is preferably at least 70%. It is more preferably 70% or more, even more preferably 80% or more, and still more preferably 90% or more. In addition, it is still more preferable that the DH/PH domain has its specific function. Further, a protein of the present invention includes a protein that is shown by an amino acid sequence with a mutation, such as deletion, substitution, addition, or the like, of one or more amino acids in the amino acid sequence set forth in SEQ ID NO: 2, and that binds to a Rho family protein. The mutation of amino acids is, for example 1 to 100, preferably 1 to 30, more preferably 1 to 20, further preferably 1 to 10, and still more preferably 1 to several in number. More preferably, it may be a protein that that has a sequence homology with the protein shown by the amino acid sequence set forth in SEQ ID NO: 2, and that binds to a Rho family protein to exhibit a GEF activity. The extent of mutation, the position of a mutation, and the like, of the amino acid are not particularly limited, as long as a protein with a mutation binds to a Rho family protein, preferably bind to a Rho family protein to exhibit a GEF activity. Still more preferably, it may be a protein having a DH/PH domain. Such a protein with a mutation may be a protein generated in nature, for example, due to mutation or post translational modification. Further, it also may be a protein prepared by introducing a mutation into a natural gene. Techniques for introducing a mutation are known in the art. For example, known gene manipulation techniques can be used for preparation. When introducing a mutation, in view of avoiding a change in the fundamental properties (such as physical properties, function, physiological activity, and immunological activity) of the protein, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived.

A protein according to the present invention further includes a protein shown by a partial sequence of the aforementioned protein. For example, a protein that is shown by a partial sequence of the protein shown by the amino acid sequence set forth in SEQ ID NO: 2, is also included in the scope of the present invention. The minimum unit of such a protein consists of consecutive amino acids, preferably 5 or more, more preferably 8 or more, even more preferably 12 or more, or 15 or more.

A protein of the present invention is preferably a human derived protein. However, the present invention includes proteins derived from mammals, for example, proteins derived from a mouse, horse, sheep, cow, dog, monkey, cat, bear, rat, rabbit, or the like, as long as the protein is a protein that has a sequence homology with the present proteins and preferably binds to a Rho family protein, and more preferably binds to a Rho family protein to exhibit a GEF activity. A suitable sequence homology with the entire amino acid sequence is normally 50% or more, preferably at least 70%. It is more preferably 70% or more, even more preferably 80% or more, and still more preferably 90% or more. Further, a protein having a DH/PH domain is more preferable for such a protein. A suitable sequence homology with the DH/PH domain is preferably at least 70%. It is more preferably 70% or more, even more preferably 80% or more, and still more preferably 90% or more.

A protein of the present invention may be a protein prepared from a cell in which a gene encoding the present protein is expressed by means of a gene manipulation technique, or from any suitable biological sample. A protein also may be a product of a cell-free synthesis system, or a chemical synthesis product. These can be subsequently further purified for use. In addition, the present protein may be a protein being expressed in a cell that contains a gene encoding the present protein. The cell may be a transformant prepared by transfecting a vector that contains a gene encoding the present protein.

A protein of the present invention can be modified to the extent that no significant functional change is involved, such as amidation of its constituent amino groups, carboxyl groups, or the like. A present protein can also be labeled with the other protein, or the like, that is added to the N-terminal or C-terminal, directly or indirectly, via a linker peptide, or the like, or by means of gene manipulation techniques, or the like. Labeling is preferably conducted in a way not to inhibit the fundamental properties of the present protein. A substance used for labeling (a labeling substance) can be exemplified by enzymes such as GST, β-Gal, HRP, ALP, or the like, tag peptides such as His-tag, Myc-tag, HA-tag, FLAG-tag, Xpress-tag or the like, fluorescent substances, such as fluorescein isothiocyanate, phycoerythrin or the like, a maltose binding protein, an immunogloblin Fc-fragment, biotin, or the like. However, it is not limited to these specific examples. Labeling can also be carried out using a radioactive isotope. One or more kinds of labeling substances in combination can be added to the present protein. These labeling substances allow the detection and/or purification of the present protein to become easier, by measuring the substance itself, or the function thereof. In addition, these substances allow, for example, the detection of the binding of the present protein to the other protein, and the measurement of the function of the present protein.

### (Method of producing a protein)

A further aspect of the present invention relates to a method of producing a protein according to the present invention. The present protein can be prepared, for example, by standard gene manipulation techniques (refer to Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.; Ulmer, K.M. Science, 1983, Vol. 219, p.666-671; Ehrlich, H. A., Ed., PCR Technology. Principles and Applications for DNA Amplification, 1989, Stockton Press and the like,) based on the nucleotide sequence information of a gene encoding the present protein. For example, at first, a cDNA library may be prepared from various kinds of cells or tissues in which the expression of the present polynucleotide was found, or cultured cells derived from these cells and tissues, in accordance with conventional methods. Then, the polynucleotide encoding the present protein may be amplified from the cDNA library, by using a primer that selectively hybridizes to the gene encoding the protein. The amplified polynucleotide may be subjected to the expression induction by using known gene manipulation techniques to produce the present protein.

Specifically, for example, the present proteins can be produced by culturing the transformants according to the present invention, and then collecting the present proteins from the culture product obtained. The transformant can be cultured according to known culture conditions and culture methods that are suitable for a host used for the preparation of the transformant. Cultivation can be carried out by employing an indicator, such as the present proteins themselves that are expressed by the transformant, or a function thereof. Alternatively, cultivation may be carried out by employing such an indicator as the present proteins themselves produced in a host or outside a host, or the amount of the protein. Otherwise, subculturing or batch culturing may be carried out by employing such an indicator as the amount of transformant in the culture medium.

When the proteins according to the present invention are expressed in a transformant, or on its cell membrane, the proteins may be extracted from the disrupted transformant. Further, when the present proteins are secreted outside the transformant, the cultured medium can be used as is, or the cultured medium can be used after removing the transformant by centrifugation or the like. As desired, the present protein can be isolated and/or purified from a cultured medium of the transformant or from the transformant.

Purification and/or isolation of the protein according to the present invention can be carried out by various isolation methods that utilize the physical properties or chemical properties of the present protein. Specific examples of the isolation methods may include ammonium sulfate precipitation, ultrafiltration, gel chromatography, ion-exchange chromatography, affinity chromatography, high performance liquid chromatography, and dialysis. These methods may be used in suitable combinations. It is preferable to employ an affinity chromatography that utilizes a column bound with a specific antibody specific to the present protein. The specific antibody to the present protein can be prepared based on the amino acid information of the protein by a well known method of producing an antibody. The purification and/or isolation may be carried out by employing an indicator to obtain the present protein, such as a function of the present protein.

The proteins of the present invention can also be produced according to conventional chemical synthesis methods. As a chemical synthesis method of a protein, for example, methods described in publications ("peptide synthesis", Maruzen Co., Ltd., 1975; and "peptide synthesis", Interscience, New York, 1996) may be used. However, it is not limited to these methods, and any known methods can be used. Specifically, .solid phase synthesis, solution phase synthesis, and the like, are known as chemical synthesis methods for proteins, and any of these methods can be used. These kinds of protein synthesis methods more specifically include a so-called stepwise elongation method that sequentially binds each amino acid, one at a time, to elongate a chain based on amino acid sequence information, and a fragment condensation method that previously synthesizes fragments consisting of several amino acids, and subsequently subjects the respective fragments to a coupling reaction. The present proteins can be synthesized by either of these methods. A condensation method used for the aforementioned protein synthesis methods can also be carried out according to conventional methods. Examples of condensation methods include an azide method, mixed anhydride method, DCC method, active ester method, oxidation-reduction method, DPPA (diphenylphosphoryl azide) method, DCC + additive (1-hydroxybenzotriazole, N-hydroxysuccinamide, N-hydroxy-5-norbomane-2,3-dicarboxyimide, and the like) method, and Woodward's method. The present protein obtained by chemical synthesis can be suitably purified in accordance with various kinds of conventional purification methods as described above.

Proteins shown by partial sequences of the proteins according to the present invention can also be obtained by cleaving the proteins according to the present invention, by a suitable peptidase.

### (Antibody)

A further aspect of the present invention relates to antibodies recognizing the proteins according to the present invention. The antibodies can be prepared using the present proteins as antigens. The antigens to be used may be the present proteins or fragments thereof. The antigen consist of amino acids of at least eight, preferably at least ten, more preferably at least twelve, and even more preferably fifteen or more amino acids. In order to prepare specific antibodies to the present proteins, it is preferable to use a region comprising a characteristic amino acid sequence of the present proteins. The amino acid sequence of this region is not necessarily required to be identical to an amino acid sequence of the present proteins or fragments thereof. An amino acid sequence of a site that is exposed outward on a tertiary structure thereof, may be preferable for such an amino acid sequence. Even if the amino acid sequence of the exposure site is not continuous on the primary structure, it is sufficient for the amino acid sequence to be continuous with respect to the exposure site. The antibodies are not particularly limited, and can be any antibody, as long as it can specifically recognize the present proteins. The phrase "specifically recognize the present proteins" means to bind to the present proteins, but not bind to or weakly bind to other proteins than the present proteins. The presence or absence of the recognition can be determined by known antigen-antibody binding reactions.

The antibodies can be produced by utilizing known antibody producing methods. For example, the antibodies can be obtained by administering to an animal an antigen alone, or an antigen bound to a carrier, with or without an adjuvant, and thereby inducing immunity, such as a humoral response, and/or a cellular response. Any known carrier can be used, as long as it does not exhibit an adverse action against the host, and is capable of enhancing the antigenicity of the antigen. Specifically, examples of the carrier include cellulose, polymeric amino acids, albumin, and keyhole limpet hemocyanin. Examples of the adjuvant include Freund's complete adjuvant (FCA), Freund's incomplete adjuvant (FIA), Ribi (MPL), Ribi (TDM), Ribi (MPL + TDM), Bordetella pertussis vaccine, muramyldipeptide (MDP), aluminium adjuvant (ALUM), and combinations of these. Mouse, rat, rabbit, goat, horse, or the like, can be preferably used as animals for immunization.

A polyclonal antibody can be acquired from the serum of an animal that was administered with an antigen by using any known method for recovering an antibody. Immunoaffinity chromatography may be employed as a preferable example of the method for recovering an antibody.

A monoclonal antibody can be produced utilizing a hybridoma that is prepared by collecting an antibody-producing cell, (for example, lymphocytes derived from spleen or lymph nodes,) from an animal that was administered with an antigen, and then fusing it with a well known immortalized cell (for example, myeloma strain such as P3-X63-Ag8 line). For example, the antibody-producing cell is fused with an immortalized cell by any known method to prepare a hybridoma which is subsequently subjected to cloning. The various cloned hybridomas are used to screen for a hybridoma that produces an antibody specifically recognizing a protein of the present invention. The antibody can be then recovered from a culture solution of that hybridoma.

A polyclonal antibody or monoclonal antibody, which is capable of recognizing or binding to a protein of the present invention, can be utilized as an antibody for purification of the present protein, reagent, labeling marker or the like. In particular, an antibody that inhibits the function of the present protein can be used for regulating the function of the present protein, and is useful for elucidating, preventing, improving, and/or treating various kinds of diseases due to an abnormality of a present protein, in amount and/or function.

### (Method of Identifying a Compound)

A still further aspect of the present invention relates to methods of identifying compounds that inhibit or enhance the function of the proteins according to the present invention, or compounds that inhibit the expression of the polynucleotides according to the present invention. Since it was found that the expression of the present polynucleotide is high in tonsil tumors such as a tonsil squamous cell carcinoma, and in bronchial tumors such as a bronchial adenoid cystic carcinoma, the present inventors believe that prevention and/or treatment of these diseases can be conducted by inhibiting the function of the present proteins and/or inhibiting the expression of the present polynucleotides. Therefore, an identification method according to the present invention may be preferably a method of identifying a compound that inhibits the function of the present proteins, or compounds that inhibit the expression of the present polynucleotides.

The identification methods according to the present invention can be carried out using at least one member selected from the proteins, the polynucleotides, the recombinant vectors, the transformants, and the antibodies which are provided in the present invention, by employing any known pharmaceutical screening system. The present identification methods include any methods that are carried out in vitro or in vivo. The present identification methods allow screening for antagonists by drug design based on the structure of the present proteins, screening for an inhibitor of the expression at the gene level by utilizing a protein synthesis system, or screening for a substance recognized by an antibody by utilizing the antibody, or the like.

A method of identifying a compound that inhibits or enhances the function of the proteins according to the present invention can be carried out using an experimental system capable of measuring the function of the present protein, which comprises allowing the present protein coexist with a compound to be tested (test compound), under conditions allowing the interaction of the present protein with the test compound, and measuring the function of the present protein, subsequently, comparing the function of the present protein in the presence of the test compound to the function of the present protein in the absence of the test compound, and finally detecting the presence, the absence, or the change of the function of the present protein, such as reduction, enhancement, disappearance, and appearance. Where the function of the present protein is reduced or eliminated in the presence of a test compound, in comparison to the function of the present protein in the absence of the test compound, it can be determined that the test compound inhibits the function of the present protein. Oppositely, where the function of the present protein is enhanced or appeared in the presence of a test compound, it can be determined that the test compound enhances the function of the present protein. The function of the proteins can be measured by direct detection of the function, or by introducing a signal as an indicator of the function into an experimental system and detecting the signal. Examples of a signal include enzymes such as GST, tag peptides such as His-tag, Myc-tag, HA-tag, FLAG-tag, or Xpress-tag, or a fluorescent protein. Any other labeling substance can be used, as long as it is used in a conventional method of identifying a compound.

The function of the proteins according to the present invention may be, for example, preferably a GEF activity for a Rho family protein. The present proteins have a DH/PH domain that is an active domain of Rho-GEF and is required for the domain for binding to a Rho family protein. Therefore, the present inventors believe that a compound that inhibits the function of the present proteins can be identified by employing an indicator, such as the binding of the present proteins to a Rho family protein.

An identification method employing such an indicator as the binding of the proteins according to the present invention to a Rho family protein, can be carried out, for example, by expressing the present proteins using gene manipulation techniques to obtain the proteins, and detecting the binding of the proteins to a Rho family protein in the presence or absence of a test compound. Specifically, for example, the present proteins can be subjected to a reaction in the presence or absence of a test compound, with a Rho family protein that is expressed as a GST-fusion protein by using gene manipulation techniques followed by binding to a glutathione-Sepharose. The identification of a compound that inhibits or enhances the binding of the present proteins to a Rho family protein can be achieved by measuring the present proteins that bind to the Rho family protein bound to a glutathione-Sepharose. Where the binding of both proteins is reduced or eliminated, in the presence of a test compound, in comparison to the binding of both proteins in the absence of the test compound, it can be determined that the test compound inhibits the binding of the present proteins to the Rho family proteins. Oppositely, where the binding of both proteins is increased in the presence of a test compound, it can be determined that the test compound enhances the binding of the present protein to the Rho family protein. The quantitative measurement of the present proteins can be carried out, for example, by using antibodies according to the present invention. An antibody labeled with a labeling substance such as enzymes (e.g. HRP or ALP), a radioactive isotope, a fluorescent substance, or biotin may be used. Alternatively, a labeled second antibody may be used. In the case of using the present proteins fused with a tag-peptide, the quantitative measurement thereof can be carried out using an antibody against the tag-peptide. Alternatively, the present proteins may be used after labeling directly with a labeling substance, such as the aforementioned enzyme, radioactive isotope, fluorescent substance, biotin, or the like. In such a case, the quantitative measurement can be carried out by measuring the labeling substance.

More specifically, an identification method employing such an indicator as the binding of the proteins according to the present invention to a Rho family protein, can be carried out using suitable cells in which a polynucleotide encoding the present protein are co-expressed with a polynucleotide encoding a Rho family protein, and using an experimental system which detects the binding of both proteins, by pull-down assay (see Example 3).

A well known two-hybrid method can be used for carrying out an identification method employing such an indicator as the binding of the proteins according to the present invention to a Rho family protein. For example, the method can be carried out wherein a plasmid for expressing a fusion protein of the present protein and a DNA binding protein, a plasmid for expressing a fusion protein of a Rho family protein and a transcription activating protein, and a plasmid containing a reporter gene that is linked to a suitable promoter gene, are introduced to a yeast, a eukaryotic cell, or the like. The identification of a compound that inhibits or enhances the binding of the present protein to a Rho family protein can be achieved by comparing the amount of expression of the reporter gene, in the presence of a test compound, to an amount of expression of the reporter gene in the absence of the test compound. In the case that the amount of expression of the reporter gene in the presence of the test compound is reduced or eliminated, compared to the amount of expression of the reporter gene in the absence of the test compound, it can be determined that the test compound inhibits the binding of the present protein to a Rho family protein. Oppositely, in the case that the amount of expression of the reporter gene in the presence of a test compound is increased, it can be determined that the test compound enhances the binding of the present protein to the Rho family protein. Any reporter genes that are used in a conventional reporter assay can be used herein. A reporter gene can be exemplified by a gene having an enzyme activity, such as, luciferase, β-Gal, chloramphenicol acetyl transferase, or the like. The expression of the reporter gene can be detected by determining the activity of the gene product, for example, an enzyme activity in the case of using the reporter gene exemplified in the above.

A surface plasmon resonance sensor, such as, the BIACORE system, or the like, can also be used in the identification method employing such an indicator as the binding of the proteins according to the present invention to a Rho family protein Alternatively, Scintillation proximity assay (SPA), or a method employing fluorescence resonance energy transfer (FRET), can also be used for carrying out the present identification method.

An identification method employing such an indicator as a GEF activity of the proteins according to the present invention for a Rho family protein, can be carried out, for example, by allowing a present protein to co-exist with a Rho family protein, which may be activated by the present protein, and measuring the amount of an activated Rho family protein in the presence or absence of a test compound. In the case that the amount of an activated Rho family protein is reduced in the presence of a test compound, compared to in the absence of the test compound, it can be determined that the test compound inhibits a GEF activity of the proteins according to the present invention for a Rho family protein. Oppositely, in the case that the amount of an activated Rho family protein is increased in the presence of a test compound, compared to in the absence of the test compound, it can be determined that the test compound enhances a GEF activity of the protein according to the present invention for a Rho family protein. An activated Rho family protein can be measured by using an antibody raised against the protein. For example, an activated Rho family protein can be measured using an effector molecule capable of binding to the activated Rho family protein, but not binding or weakly binding to the non-activated Rho family protein, by effector pull down assay.

Specifically, a Rho family protein activated by the proteins according to the present invention, can be quantitatively measured using suitable cells in which the polynucleotide encoding the protein according to the present invention was co-expressed with a polynucleotide encoding a Rho family protein. An activated Rho family protein can be quantitatively measured by utilizing a GST-fused effector molecule fragment containing a binding site of the protein, by effector pull down assay. Specifically, an activated Rho family protein is first subjected to a reaction with a GST-fused effector molecule fragment. After that, the effector molecule fragment is collected by using anti-GST-tag antibody. Then, the activated Rho family protein bound to the collected effector molecule fragment is quantitatively measured. An amount of the activated Rho family protein can be measured by electrophoresis and Western blotting. An effector molecule that binds to an activated Rho family protein is different depending on the type of a Rho family protein. Therefore, a suitable effector protein may be selected for use according to the kind of a Rho family protein used. For example, activated RhoA is known to bind to the effector molecule, Rhotekin. Therefore, Rhotekin may be a suitable effector molecule for use to identify a compound that inhibits or enhances the activation of RhoA.

An identification method employing such an indicator as a GEF activity of the proteins according to the present invention for a Rho family protein, can be also carried out by allowing the present proteins to co-exist with a Rho family protein, which is activated by the present proteins and is bound to radioactive isotope-labeled GDP, and with GTP, and subsequently measuring the amount of an activated Rho family protein in the presence or absence of a test compound. The activated Rho family protein can be quantitatively determined by measuring the reduced amount of the Rho family protein being bound to radioactive isotope-labeled GDP. Such a method can be carried out by referring a method described in a known literature (Journal of Biological Chemistry, 1996, Vol. 271, No. 44, p. 27374-27381).

The phrase "inhibiting a GEF activity for a Rho family protein" means to inhibit the conversion of a GDP-bound inactive form of Rho family protein to a GTP-bound active form of Rho family protein. More specifically, it means to reduce or to delete the amount of a GTP-bound active form of Rho family protein. The phrase "enhancing a GEF activity for a Rho family protein" means to enhance the conversion of a GDP-bound inactive form of Rho family protein to a GTP-bound active form of Rho family protein. More specifically, it means to increase the amount of a GTP-bound active form of Rho family protein.

A Rho family protein used in the identification method according to the present invention may be a protein lacking of a part thereof or a protein labeled with an aforementioned labeling substance, as long as the activation thereof by a protein according to the present invention is not affected. The labeling substance is exemplified in the above description.

An identification method employing such an indicator as the expression of a polynucleotide according to the present invention, can be carried out using an experimental system capable of measuring the expression of the present polynucleotide, which comprises allowing the present polynucleotide co-exist with a test compound under conditions allowing the interaction of the present polynucleotide with the test compound, and measuring the expression of the present polynucleotide. Subsequently, the expression of the present polynucleotide in the presence of the test compound can be compared to the expression of the present polynucleotide in the absence of the test compound, for detecting the presence, the absence, or the change of the expression of the present polynucleotide, such as reduction, enhancement, disappearance, and appearance. In the case that the expression of the present polynucleotide is reduced or disappeared in the presence of a test compound in comparison to the expression of the present polynucleotide in the absence of the test compound, it can be determined that the test compound inhibits the expression of the present polynucleotide. Oppositely, in the case that the expression of the present polynucleotide is increased in the presence of a test compound, it can be determined that the test compound enhances the expression of the present polynucleotide.

Specifically, for example, the identification method employing such an indicator as the expression of a polynucleotide according to the present invention, can be carried out using an experimental system using the transformant according to the present invention, for expressing the present polynucleotide, which comprises contacting the transformant with a test compound, and then measuring the expression of the present polynucleotide. The expression of the present polynucleotide can be measured easily by detecting the amount of expressed protein, or by detecting the function of the protein. Further, the expression of the present polynucleotide can be measured also by introducing, for example, a signal as an indicator of the expression into the experimental system, and detecting the signal. Examples of a signal include: enzymes such as GST; tag peptides such as His-tag, Myc-tag, HA-tag, FLAG-tag, or Xpress-tag; or a fluorescent substance. A method of detecting these signals is well known to those skilled in the art.

An identification method employing such an indicator as the expression of a polynucleotide according to the present invention, can also be carried out by, for example, preparing a vector that comprises a promoter region of a gene corresponding to the present polynucleotide and a reporter gene linked downstream of the promoter region instead of the present polynucleotide, and contacting a cell, e.g. a eukaryotic cell, which contains the vector, with the test compound, and then determining the presence or absence of, or a change in expression of the reporter gene. Any reporter genes that are used in a conventional reporter assay can be used herein. Specifically, a reporter gene can be exemplified by a gene having an enzyme activity, such as, luciferase, β-Gal, chloramphenicol acetyl transferase, or the like. The expression of the reporter gene can be detected by determining the activity of the gene product, for example, an enzyme activity in the case of using a reporter gene exemplified above.

### (Compound)

A compound obtained by an identification method according to the present invention can be utilized as a candidate compound for an inhibitor of the expression of a polynucleotide according to the present invention, an inhibitor or an antagonist of the function of a protein according to the present invention. For example, the compound can be utilized as a candidate compound for an inhibitor of a GEF activity for a Rho family protein, namely, an inhibitor of an activation of a Rho family protein. A compound obtained by the present identification method can be utilized as a candidate compound for an agent for enhancing the expression of the polynucleotide according to the present invention, or an agent for enhancing the function of the protein according to the present invention. Since it was found that the expression of the present polynucleotide is high in tonsil tumors such as a tonsil squamous cell carcinoma, and in bronchial tumors such as an bronchial adenoid cystic carcinoma, the present inventors believe that prevention and/or treatment of the diseases can be conducted by inhibiting the function of the present protein and/or inhibiting the expression of the present polynucleotide. Therefore, a compound obtained by the identification method according to the present invention may be preferably a compound that inhibits the function of the present protein and/or a compound that inhibits the expression of the present polynucleotide. These candidate compounds can be prepared as a medicament by taking into consideration the balance between usefulness and toxicity. Such a medicament is useful for preventing and/or treating various kinds of symptoms due to an abnormality in the function of the present protein and/or an abnormality in the expression of the present polynucleotide. The compounds according to the present invention include compounds that are obtained by other methods than the present identification methods if they are capable of inhibiting the function of the present protein and/or the expression of the present polynucleotide, or are capable of enhancing the function of the present protein and/or the expression of the present polynucleotide.

### (Pharmaceutical Composition)

A further aspect of the present invention relates to a medicament or a pharmaceutical composition which is based on inhibiting or antagonizing the function of a present protein and/or the expression of a present polynucleotide, or which is based on enhancing the function of the present protein and/or the expression of the present polynucleotide. The medicament or the pharmaceutical composition contains a protein, a polypeptide, a recombinant vector, a transformant, an antibody, or a compound, which is provided according to the present invention, as an active ingredient.

A medicament according to the present invention can be a medicament that contains an effective amount of at least one member selected from a protein, a polynucleotide, a recombinant vector, a transformant, an antibody, or the compound, which is provided according to the present invention, as an active ingredient. In general, it is preferable to prepare a pharmaceutical composition using one or more kinds of pharmaceutically acceptable carriers (pharmaceutical carriers).

An amount of the active ingredient contained in the pharmaceutical composition according to the present invention can be suitably selected from a wide range. In general, a suitable amount may fall within a range of approximately 0.00001 to 70 wt%, preferably approximately 0.0001 to 5 wt%.

A pharmaceutical carrier may be a diluent or excipient, which can be generally used in accordance with the form of use of the pharmaceutical composition, such as, a filler, an extender, a binder, a wetting agent, a disintegrator, and/or a lubricant. These can be suitably selected and used in accordance with the form of use of the pharmaceutical composition used.

The pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be suitably selected, and used in accordance with the form of use of a pharmaceutical composition of the present invention.

As desired, various ingredients used in conventional protein preparations can be suitably used herein, such as, a stabilizer, a bacteriocide, a buffer agent, an isotonizing agent, a chelating agent, a pH adjuster, or a surfactant, for preparing the pharmaceutical composition.

As a stabilizer, the following may be used: human serum albumin, common L-amino acids, sugars, and cellulose derivatives. These can be used independently or in combination with a surfactant, and the like. Use of these in such a combination may give increased stability to an active ingredient. An L-amino acid is not particularly limited, and may be any one of glycine, cysteine, glutamic acid, and the like. A sugar is not particularly limited, and may be any one of the monosaccharides (such as glucose, mannose, galactose, and fructose), sugar alcohols (such as mannitol, inositol, and xylitol), disaccharides (such as sucrose, maltose, and lactose), polysaccharides (dextran, hydroxypropylstarch, chondroitin sulfate, and hyaluronic acid), derivatives thereof, and so on. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like.

A surfactant is not particularly limited, and can be both an ionic surfactant and/or a non-ionic surfactant. As a surfactant, the following may be used: polyoxyethyleneglycol sorbitan alkyl ester base; polyoxyethylene alkyl ether base; sorbitan monoacyl ester base; or a fatty acid glyceride base.

As a buffer agent, the following may be used: boric acid; phosphoric acid; acetic acid; citric acid; ε-aminocaproic acid; glutamic acid; and/or a salt thereof, for example, an alkali metal salt and/or an alkaline earth metal salt, such as a sodium salt, a potassium salt, a calcium salt and a magnesium salt.

As an isotonizing agent, the following may be used: sodium chloride; potassium chloride; sugars; or glycerin.

As an isotonizing agent, the following may be used: sodium chloride; potassium chloride; sugars; or glycerin.

As a chelating agent, sodium edentate and citric acid may be used.

The medicaments and the pharmaceutical compositions according to the present invention can be used as solution preparations. Alternatively, they can be freeze-dried, so as to be in a good state for preservation. They can be used by dissolving them in water, a buffered solution containing saline, and the like, and then adjusting them to a suitable concentration, at the time of use.

The medicaments and the pharmaceutical compositions according to the present invention can be used as agents for preventing and/or treating a disease due to an abnormality in the function of a present protein and/or an abnormality in the expression of a present polynucleotide. In addition, the medicaments and the pharmaceutical compositions can be used in methods for preventing and/or treating the disease.

For abnormal symptoms due to an excess of a function of a protein according to the present invention, and/or the expression of a polynucleotide according to the present invention, an effective amount of an inhibitor that inhibits the function of a present protein and/or the expression of a present polynucleotide, may be administered to a subject together with a pharmaceutically acceptable carrier. The administration may result in obtaining an effect such as prevention, improvement, or treatment of the abnormal symptoms. Alternatively, the similar effect can be obtained by inhibiting the intrinsic expression of a present polynucleotide using an expression block method. The inhibition of the expression of a present polynucleotide can be achieved, for example, by using an anti-sense oligonucleotide, such as an oligonucleotide consisting of a partial sequence of a present polynucleotide. An oligonucleotide corresponding to a non-coding region of a present polynucleotide as well as an oligonucleotide corresponding to a coding region thereof, is useful as an anti-sense oligonucleotide used herein. In order to specifically inhibit the expression of a present polynucleotide, it is preferable to use a nucleotide sequence of a characteristic region of the polynucleotide.

A disease due to an abnormality in the function of a present protein and/or an abnormality in the expression of a present polynucleotide may be, for example, a tumor disease preferably exemplified by tonsil tumors and bronchial tumors. The tissue distribution of the polynucleotide shown by the nucleotide sequence set forth in SEQ ID NO: 1, which is a specific example of a polynucleotide according to the present invention, was found to be 2.77 times higher in a tonsil squamous cell carcinoma that is one of tonsil tumors, as compared to that in a normal tonsil tissue. Further, the expression of the polynucleotide was also found to be 1.65 times higher in a bronchial adenoid cystic carcinoma, as compared to that in a normal bronchial tissue. As described above, the protein encoded by the polynucleotide shown by the nucleotide sequence set forth in SEQ ID NO: 1 binds to a Rho family protein. Therefore, the protein can be considered to work as Rho-GEF. Among the Rho-GEF genes isolated so far, the following genes are known to relate to cancer: vav (Non-Patent References 3 and 4); ost (Non-Patent Reference 5); ibc (Non-Patent Reference 6) and the like. Thus, the present inventors believe that the high expression of the present polynucleotides relates to a tumor disease such as tonsil tumors and bronchial tumors. Further, the present inventors believe that the prevention and/or treatment of these diseases can be conducted by inhibiting the function of the present proteins and/or the expression of the present polynucleotides. The medicaments and the pharmaceutical compositions according to the present invention may be useful as agents for preventing and/or treating a tumor disease such as tonsil tumors and/or bronchial tumors. In addition, the medicaments and the pharmaceutical compositions may be used in methods of preventing and/or treating a tumor disease such as tonsil tumors and/or bronchial tumors.

Suitable dosage ranges of the medicament and the pharmaceutical composition according to the present invention are not particularly limited, and can be determined in accordance with the following: effectiveness of the ingredients contained therein; the administration form; the route of administration; the type of disease; the characteristics of the subject (e.g., body weight, age, symptomatic conditions, and whether a subject is taking other pharmaceutical agents); and the judgment of a physician in charge. In general, a suitable dosage may fall, for example, within a range of about 0.01 µg to 100 mg, per 1 kg of the body weight of the subject, and preferably within a range of about 0.1 µg to 1 mg, per 1 kg of body weight. However, the dosage may be altered using conventional experiments for optimization of a dosage that are well known in the art. The aforementioned dosage can be divided for administration once to several times a day. Alternatively, periodic administration once every few days or few weeks can be employed.

When administering the medicament or the pharmaceutical composition according to the present invention, the medicament or the pharmaceutical composition may be used alone, or may be used together with other compounds or medicaments useful for preventing and/or treating the target disease.

In terms of a route of administration, it may be either systemic administration or local administration. The route of administration that is appropriate for a particular disease, symptomatic condition, or other factors, should be selected. For example, parenteral administration including normal intravenous injection, intra-arterial administration, subcutaneous administration, intracutaneous administration, and intramuscular administration can be employed. Oral administration can be also employed. Further, transmucosal administration or dermal administration can be employed. In the case of use for tumor disease, it may be preferable to employ a direct administration into the tumor by injection, and the like.

In terms of an administration form, various forms can be selected in accordance with a treatment purpose. For example, a solid formulation may be employed such as a tablet, pill, powder, powdered drug, fine granule, granule, or a capsule. Alternatively, a liquid formulation can be employed such as an aqueous formulation, ethanol formulation, suspension, fat emulsion, liposome formulation, clathrate such as cyclodextrin, syrup, or an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation, and the like, which can be respectively blended, formed and prepared according to conventional methods.

### (Diagnosing Method)

The proteins, polynucleotides, recombinant vectors, transformants, antibodies, or the compounds, which are provided in the present invention, can be used by themselves as a means for diagnosing a disease, such as a diagnostic marker or a diagnostic reagent.

According to the present invention, for example, use of all or a part of a polynucleotide according to the present invention allows the specific detection of the presence or absence of an abnormality in a polynucleotide or a gene containing the polynucleotide, or the presence or absence of expression thereof, in an individual, or in various kinds of tissues. The detection of a polynucleotide according to the present invention allows for a diagnosis of susceptibility to, onset of, and/or prognosis of, a disease due to an abnormality in the amount of a polynucleotide or a gene containing the polynucleotide, and/or, an abnormality in the function thereof.

Diagnosis of a disease can be carried out, for example, by detecting the presence of a polynucleotide according to the present invention, by determining the existing amount thereof, and/or by identifying a mutation, with respect to a sample to be tested (test sample). In comparison to a normal control sample, a change in the existence of a present polynucleotide, and a quantitative change thereof, can be detected. Alternatively, an amplified product obtained by amplifying a present polynucleotide using a known method may be subjected, for example, to the measurement of a change in size. In comparison to a normal genotype, a mutation such as deletion or insertion can be detected. Further, a polynucleotide amplified from a test sample may be subjected, for example, to hybridization to a labeled polynucleotide according to the present invention, which allows the isolation of a point mutation. The detection of such a mutation allows the aforementioned diagnosis.

The present invention can provide a qualitative or quantitative measurement method, for a polynucleotide according to the present invention, in a test sample. Further, a qualitative or quantitative measurement method for the mutation, in the specific region of a polynucleotide, can also be provided.

The tissue distribution of the polynucleotide shown by the nucleotide sequence set forth in SEQ ID NO: 1, was found to be 2.77 times higher in a tonsil squamous cell carcinoma that is one of tonsil tumors, as compared to that in a normal tonsil tissue. The tissue distribution was examined using BioExpress (GeneLogic), disease database information. Further, the expression of the polynucleotide was also found to be 1.65 times higher in a bronchial adenoid cystic carcinoma, as compared to that in a normal bronchial tissue. Meanwhile, as described in the above, the present inventors considered that the high expression of a present polynucleotide relates to a tumor disease such as tonsil tumors and bronchial tumors. Therefore, the detection of the increased amount of expression of the polynucleotide in a test sample allows execution of a method of determining whether the test sample is a test sample that is derived from a tumor disease such as tonsil tumors and bronchial tumors, or not. Such a determination method may also be included in the scope of the present invention. In this determination method, the increased amount of expression of the polynucleotide can be detected by comparing a test sample to a normal control sample. A human tonsil-derived tissue, or a human bronchia-derived tissue, may be preferably used as a test sample. A normal human tonsil -derived tissue, or a normal human bronchia-derived tissue, may be preferably used as a control sample. In the case that the amount of expression of the polynucleotide is increased in a sample derived from human tonsil tissue, compared to that in a control sample, preferably at least 2.5 times or more, more preferably at least 3 times or more, it can be determined that the test sample is a human tonsil tumor-derived sample. In the case that the amount of expression of the polynucleotide is increased in a sample derived from human bronchial tissue, compared to that in a control sample, preferably at least 1.5 times or more, more preferably at least 2 times or more, it can be determined that the test sample is a human bronchial tumor-derived sample. The phrase "amount of expression of the polynucleotide according to the present invention" means the amount of the transcription product of the polynucleotide.

A test sample is not limited to a human tonsil-derived tissue, or a human bronchia-derived tissue. Any tissues and cells derived from a living body can be used. Specifically, a sample derived from a living organism such as a cell, blood, urine, saliva, spinal fluid, biopsy tissue, or autopsy material, and the like, may be used as a test sample. As desired, a nucleic acid may be extracted from a test sample to prepare a nucleic acid sample for use. A nucleic acid may be any of a genomic DNA, RNA, and cDNA. A nucleic acid also may be enzymatically amplified by employing PCR, or other amplification methods. A nucleic acid sample may also be prepared according to various methods, for facilitating detection of a target sequence, for example, denaturation, digestion with restriction enzymes, electrophoresis, or dot blotting.

Any known gene detection methods can be used for detecting a polynucleotide according to the present invention or a gene containing the polynucleotide. Specifically, for example, plaque hybridization, colony hybridization, Southern blotting, Northern blotting, the NASBA method, reverse transcription-polymerase chain reaction (RT-PCR), or the like can be used. In addition, a gene detection method which allows cell level measurement, such as in situ RT-PCR, in situ hybridization, or the like, can be used for the detection. In such a gene detection method, it is useful to use an oligonucleotide, which consists of a partial sequence of a present polynucleotide, and has the property as a probe or a primer, for carrying out the isolation and/or the amplification of the polynucleotide, a gene containing the polynucleotide, or a mutant gene thereof. The phrase "oligonucleotide having the property as a probe" means an oligonucleotide that is capable of specifically hybridizing only to the present polynucleotide, and consists of a characteristic sequence of a present polynucleotide. The phrase "oligonucleotide having the property as a primer" means an oligonucleotide that is capable of specifically amplifying only a present polynucleotide, and consists of a characteristic sequence of a present polynucleotide. Further, when detecting a mutant gene capable of being amplified, a primer or a probe having a sequence with a predetermined length, which contains a mutation site within the gene, is prepared and used. A probe and a primer may have a nucleotide sequence consisting of, preferably from about 5 to 50 nucleotides, more preferably from about 10 to 35 nucleotides, and even more preferably from about 15 to 30 nucleotides. Specifically, an oligonucleotide shown by the nucleotide sequence set forth in SEQ ID NO: 5, or SEQ ID NO: 6, can be preferably used as a primer for amplifying a polynucleotide of the present invention or a fragment thereof, or as a probe for detecting a present polynucleotide. A labeled probe is normally used as the probe, but the unlabeled prove can also be used. Alternatively, the detection can also be carried out by measuring the specific binding to a ligand that was labeled directly or indirectly. Various methods are known for labeling a probe and a ligand. For example, nick translation, random priming, or a method utilizing kinase treatment, may be used. Labeling substances suitable for use include a radioactive isotope, biotin, a fluorescent substance, a chemiluminescent substance, an enzyme, an antibody, and the like.

PCR is preferable as a gene detection method, from the viewpoint of sensitivity. Any well-known method of PCR can be employed, as long as it is a method that uses a primer capable of specifically amplifying a polynucleotide according to the present invention, a gene containing the polynucleotide, or a mutant gene thereof. For example, RT-PCR may be employed. In addition, various modified PCT methods used in the art can be applied.

In addition to detection of a gene, PCR allows quantitative measurement of a polynucleotide according to the present invention, a gene containing the polynucleotide, or a mutant gene thereof. Such an assay method may be exemplified by a competitive assay, such as, an MSSA method, or PCR-SSCP, which is known as a mutation detection method that utilizes a change in mobility accompanying a structural change of a single-stranded DNA.

According to the present invention, for example, use of a protein according to the present invention allows the specific detection of, the presence or absence of, an abnormality in the protein itself, and in its function, in an individual or in various kinds of tissues. The detection of an abnormality in a protein according to the present invention, and in its function, allows a diagnosis of susceptibility to, onset of, and/or prognosis of, a disease due to an abnormality in the amount of the protein and/or an abnormality in its function.

The diagnosis of a disease by detecting a protein can be carried out, for example, by detecting the presence of a protein, by determining the existing amount thereof, and/or by identifying a mutation, with respect to a sample to be tested (test sample). That is to say, a protein according to the present invention, and/or its mutant, may be quantitatively or qualitatively measured. In comparison to a normal control sample, a change in the existence of a present protein, and a quantitative change thereof, can be detected. Alternatively, in comparison to a normal control sample, a mutation can be detected, for example, by determining an amino acid sequence. The detection of such a change or a mutation allows the aforementioned diagnosis. A test sample is not limited so long as the sample has the present protein and/or its mutant. A biological sample derived from a living organism, such as blood, serum, urine, biopsy tissue, and the like, may be used as a test sample.

A protein according to the present invention, and the protein with a mutation, can be measured using a protein according to the present invention, a fragment thereof, or an antibody against the protein or the fragment. Specifically, for example, the protein shown by the amino acid sequence set forth in SEQ ID NO: 2, a protein shown by the amino acid sequence having a deletion, substitution, insertion, or addition of one or several or more amino acids, in the amino acid sequence of the protein, the fragment thereof, or an antibody against the protein or the fragment, can be used.

Any protein detection methods, or protein quantitation methods which are well known in the art, can be used for quantitative or qualitative measurement of the protein. For example, the amino acid sequence analysis of a present protein allows a detection of a mutant protein. More preferably, an antibody (a polyclonal antibody or a monoclonal antibody) may be used for detecting the difference in the protein sequence, or the presence or absence of the protein.

The present invention includes a qualitative or quantitative measurement method for a present protein in a test sample, or a qualitative or quantitative measurement method for a mutation in the specific region of the protein.

Specifically, the aforementioned detection may be carried out by subjecting a test sample to immunoprecipitation, using a specific antibody raised against a present protein, and then analyzing the present protein by Western blotting or immunoblotting. Further, the detection of a present protein in a paraffin tissue section, or a frozen tissue section, may be carried out by means of immuno-histochemical techniques using a specific antibody raised against the present protein.

The preferable methods of detecting a present protein or its mutant, may be, for example, enzyme-linked immunosorvent assay (ELISA), radio immuno assay (RIA), immunoradiometric assay (IRMA), and immunoenzymometric assay (IEMA), including a sandwich method using a monoclonal antibody and/or a polyclonal antibody. Alternatively, radio immuno assay, competitive binding assay and the like may be employed.

A protein, polynucleotide, recombinant vector, transformant, and/or antibody, which are provided according to the present invention, can each be used by themselves or in combination as a reagent, or the like. The reagent may be contained in a substance such as a buffer solution, a salt, a stabilizer, and/or an antiseptic agent, in addition to at least one member selected from a protein, a polynucleotide, a recombinant vector, a transformant, and an antibody which are provided according to the present invention. A known formulation means may be introduced, in accordance with the respective properties, at the time of formulation. The reagent can be used, for example, in a determination method, a method of identifying a compound, or a method of measuring the present protein, or a present polynucleotide, which is provided according to the present invention. In addition, the reagent is useful, for example, in elucidating an intracellular signal transduction pathway wherein a protein or a polynucleotide according to the present invention may be involved and the reagent can be used in fundamental research, such as research for a disease due to an abnormality of the protein or the polynucleotide.

The present invention further provides a reagent kit containing at least one member selected from a protein, a polynucleotide, a recombinant vector, a transformant, and an antibody which are provided according to the present invention. The kit may further contain a substance necessary for carrying out a measurement, such as a labeling substance, for detecting a protein or a polynucleotide according to the present invention, an agent for detecting the labeling substance, a reaction diluent, a standard antibody, a buffer solution, a washing agent, and a reaction terminating solution. As a labeling substance, the proteins described above, radioactive isotype, or the like, can be used. A labeling substance may be previously linked to the protein or the polynucleotide according to the present invention. The present reagent kit can be used, for example, in the determination method, the method of identifying a compound, or the method of measuring a present protein, or a present polynucleotide, which is provided according to the present invention. In addition, the present reagent kit can also be used as a test agent or a test kit in the test method using the aforementioned measurement method. The present reagent kit can further be used as a diagnostic agent, or a diagnostic kit in the diagnostic method using the aforementioned measurement method.

Hereinafter, the present invention may be explained more specifically with the following examples. However, the aspect of the present invention is not limited to these examples.

### Example 1

### (Gene Cloning)

A human spleen-derived cDNA library was constructed in accordance with a method of Ohara et al. (Ohara, O. et al., DNA research, 1997, Vol. 4, p. 53-59). Specifically, double strand cDNA was synthesized by using a Superscript II reverse transcriptase kit (GIBCO BRL). At that time, an oligonucleotide (GACTAGTTCTAGATCGCGAGCGGCCGCCC(T)15: SEQ ID NO: 12, GIBCO BRL) which has a NotI site was used as a primer, and a human spleen mRNA (Clontech: catalogue number 6542-1) was used as a template. The cDNA was ligated to an adaptor (GIBCO BRL) which has a SalI site, and then digested with NotI, followed by electrophoresis with 1 % concentration of a low melting point agarose to purify DNA fragments of 3 kb or more. The purified cDNA fragment was ligated with pBluescriptII SK+ plasmid treated with SalI-NoI restriction enzyme. The recombinant plasmid was introduced into E. coli. ElectroMax DH10B strain (GIBCO BRL) by electroporation. Approximately 10,000 recombinants were selected from the constructed cDNA library to determine the both terminal DNA sequences of these clones. Among them, approximately 420 clones which contain novel genes were selected and determined for the entire nucleotide sequences of the cDNAs. Sequencing was carried out using DNA sequencer (RISA: SHIMADZU) and a reaction kit purchased from PE Applied Biosystem. The most of the sequences of shotgun clones was determined by using a dye terminator method (terminator labeling method).

The ORF was predicted for cDNA clones, whose entire nucleotide sequences were determined, by a conventional analysis method using a computer program. Subsequently, the ORF region was analyzed by a domain motif search, to identify a cDNA containing a region encoding a DH/PH domain that is an active domain of Rho-GEF.

As a result, cDNA having a novel nucleotide sequence, and containing a DH/PH domain coding region, was identified. Hereinafter, the cDNA is referred to as sh06537.

A sequence analysis showed that sh06537 consists of the nucleotide sequence set forth in SEQ ID NO: 1 (5541 bp long), and encodes the amino acid sequence set forth in SEQ ID NO: 2 (1597 amino acids). A DH domain coding region of sh06537 corresponds to the region consisting of the nucleotides from the 3706^{th} nucleotide to the 4245^{th} nucleotide of the nucleotide sequence set forth in SEQ ID NO: 1. A PH domain coding region corresponds to the region consisting of the nucleotides from the 4345^{th} nucleotide to the 4680^{th} nucleotide of the nucleotide sequence set forth in SEQ ID NO: 1. In addition, a DH domain of the protein encoded by sh06537 corresponds to 180 amino acid resides from the 1178^{th} valine (Val) to the 1357^{th} asparagine (Asn) of the amino acid sequence set forth in SEQ ID NO: 2. A PH domain corresponds to 112 amino acid resides from the 1391^{st} leucine (Leu) to the 1502^{nd} glutamic acid (Glu) of the amino acid sequence set forth in SEQ ID NO: 2.

### Example 2

### (DNA expression and purification)

An expression vector for sh06537 that was isolated in Example 1 was prepared using GATEWAY^{™} Cloning Technology (Invitrogen). Then, the expression vector was used for expressing a protein that is encoded by sh06537, as FLAG-tagged protein, in 293EBNA cells (Invitrogen). In addition, an expression vector for DNA (hereinafter, may be referred to sh06537-D), which consists of a partial nucleotide sequence of sh06537 and lacks a DH/PH domain coding region, was prepared in the same manner. Then, the expression vector was used for expressing a protein, which consists of a partial amino acid sequence of a protein encoded by sh06537 and lacks a DH/PH domain, as FLAG-tagged protein, in 293EBNA cells, as well. The expression was confirmed by Western blotting.

Specifically, sh06537 that was isolated in Example 1 and inserted into the SalI-NotI site of pBluescriptII SK+, was used as a template for amplifying the ORF region, without a stop codon, of sh06537. Amplification was carried out by using pfu turbo (STRATAGENE). Oligonucleotides shown by the respective nucleotide sequences set forth in SEQ ID NO: 5 and SEQ ID NO: 6, were used as amplification primers. Then, an amplification product was inserted into pENTR/SD/D-TOPO in a reaction using TOPO cloning system to prepare an entry vector. The prepared entry vector was cleaved with HindIII/EcoRI to produce a gene fragment. On the other hand, sh06537 was cleaved with HindIII/EcoRI/ScaI to produce a gene fragment. These gene fragments were ligated to each other, and introduced into competent cells (TOP10: Invitrogen). The transformed E. Coli cells were used for purifying DNA (herein after, may be referred to sh06537-F), by using a purification kit (QIAGENE). Sequencing analysis confirmed a sequence of the amplified region, and sequences upstream and downstream of the nucleotide sequence after treated with restriction enzymes, to be correct. Sequencing reaction was carried out using DYEnamic ET Terminator Cycle Sequencing Kit (Amersham Biosciences). Electroporation and analysis were carried out using ABI PRISM 377. Next, sh06537-F was used together with a C-terminal 3x FLAG tagged protein-expression vector that was cleaved with BspEI for preparing a sh06537-F expression plasmid in a recombination reaction using LR clonase enzyme. Use of this expression plasmid allows the production of a protein encoded by sh06537-F, as a C-terminal 3x FLAG-tagged protein.

Next, an expression vector for sh06537-D was constructed using GATEWAY^{™} Cloning Technology (Invitrogen). Specifically, at first, sh06537, that was isolated in Example 1 and inserted into the SalI-NotI site of pBluescriptII SK+, was used as a template for amplifying a partial sequence of sh06537. The partial sequence of sh06537 consists of nucleotides from the 175^{th} nucleotide to the 3693^{rd} nucleotide of SEQ ID NO: 1 (i.e., SEQ ID NO: 3), and lacks a DH/PH coding region. The amplification was carried out using pfu turbo (STRATAGENE), and using oligonucleotides shown by the respective nucleotide sequences set forth in SEQ ID NO: 5 and SEQ ID NO: 7 as primers. Then, an amplification product was inserted into pENTR/SD/D-TOPO in a reaction using TOPO cloning system to prepare an entry vector. The prepared entry vector was cleaved with NaeI/EcoRI/PmaCI to produce a gene fragment. On the other hand, sh06537 obtained in Example 1 was cleaved with NaeI/EcoRI/SalI to produce a gene fragment. These gene fragments were ligated to each other and introduced into competent cells (TOP10: Invitrogen). The transformed E. Coli cells were used for purifying DNA using a purification kit (QIAGEN). Sequencing analysis confirmed a sequence of the amplified region and sequences upstream and downstream of the nucleotide sequence after treated with restriction enzymes, to be correct. Sequencing reaction was carried out using DYEnamic ET Terminator Cycle Sequencing Kit (Amersham Biosciences). Electroporation and analysis were carried out using ABI PRISM 377. Next, the purified DNA was used together with a C-terminal 3x FLAG-tagged protein-expression vector that was cleaved with BspEI, for preparing a sh0537-D expression plasmid, in a recombination reaction using LR clonase enzyme. Use of this expression plasmid allows the production of a protein encoded by sh06537-D as a C-terminal 3x FLAG-tagged protein.

The sh06537-F expression vector and the sh06537-D expression vector were respectively transfected into 293EBNA cells by lipofection. The 293EBNA cells had been seeded the day before transfection at 6 x 10⁴ cells/well in 24 well plates, and cultured in culture medium (IMDM medium (SIGMA), 10 % fetal bovine serum, 4 mM glutamine, and 10 µg/mL gentamycin). On the next day, the transfection was carried out by means of using Lipofectamine^{™} 2000 (Invitrogen). Specifically, at first, each expression vector was added to serum-free DMEM (SIGMA), subsequently mixed with DMEM containing Lipofectamine^{™} 2000, and incubated at room temperature for 20 minutes. The obtained mixture was added to 293EBNA cells that had been seeded the day before, and cultured at 37 °C in the presence of 5% CO₂. The cells subjected to transfection were further cultured at 37 °C in the presence of 5% CO₂. On two days after transfection, the culture medium was removed from cultured cells to wash the cells with phosphate-buffered physiological saline (PBS). Then, the cells were lysed with lysis buffer containing 1% protease inhibitor cocktail (SIGMA) to prepare a cell lysate. The lysis buffer was composed of 25 mM Tris-HCl (pH 7.5), 150 mM NaCl, 1 mM CaCl₂, and 1% Triton X-100.

Each cell lysate was mixed with an equal volume of SDS-PAGE sample buffer, and subjected to heat treatment at 100 °C for 5 minutes, to prepare a sample for electrophoresis. SDS-polyacrylamide gel electrophoresis (SDS-PAGE) was carried out, and then the gel was equilibrated for 5 minutes or more, in blotting buffer. The proteins were transferred onto PVDF membrane. After blotting, the PVDF membrane was subjected to blocking at 4 °C for overnight in a solution (TBS-T+BA) containing the TBS-T mixed with Block Ace (Dainippon Pharmaceutical) at a ratio of 3:1. After blocking, the PVDF membrane was washed with TBS-T for 10 minutes or more with shaking. As a SDS-PAGE sample buffer, β-ME Sample Treatment for Tris SDS (Daiichi Pure Chemical s) was used. The running buffer for SDS PAGE was composed of 100 mM Tris, 192 mM glycine, 0.1% SDS, pH 8.3 (Bio Rad). The blotting buffer is composed of 25 mM Tris, 40 mM ε-amino-n-caproic acid, 20% methanol, and 0.05% SDS. The TBS-T is composed of 150 mM NaCl, 10 mM Tris-HCl (pH 7.5), and 0.05% Tween-20.

Anti-FLAG M2 monoclonal antibody (SIGMA) that was diluted to 1000-fold with TBS-T+BA was added to the PVDF membrane, and incubated at 37 °C for 1 hour or more. After that, the PVDF membrane was washed three times with TBS-T with shaking (20 minutes or more for each wash), and HRP-labeled goat anti-mouse IgG antibody (Cell Signaling Technology), that was diluted to 1000-fold with TBS-T+BA was added to the membrane. The membrane was incubated at 37 °C for 1 hour or more. Finally, the PVDF membrane was washed three times with TBS-T with shaking (20 minutes or more for each wash), followed by detection of the expressed protein that reacted with anti-FLAG antibody by using ECL Plus Western Blotting Detection System (Amersham biosciences). The chemiluminescence was visualized with detection device, Lumino Imaging Analyzer (TOYOBO).

The cell lysate prepared from the cells that were transfected with the sh06537-F expression vector showed a major band around 180 kDa. In addition, the cell lysate prepared from the cells that were transfected with the sh06537-D expression vector showed a major band around 130 kDa. The deduced molecular weight of a protein, which is expressed as a FLAG-tagged protein by the sh06537-F expression vector, is approximately 180 kDa. The deduced molecular weight of a protein, which is expressed as a FLAG-tagged protein by the sh06537-D expression vector, is approximately 180 kDa. On the other hand, none of these bands was detected in the cell lysate prepared from cells that were added with Lipofectamine^{™} 2000 without transfecting with the vector. Therefore, the major bands observed are believed to be a protein encoded by sh06537-F and a protein encoded by sh06537-D, respectively. Thus, the protein encoded by sh06537-F and the protein encoded by sh06537-D were obtained.

### Example 3

### (Detection of the binding of a protein encoded by sh06537-F to a Rho family protein)

The binding of a protein encoded by sh06537-F to a Rho family protein was examined by pull down assay, using the sh06537-F expression vector constructed in Example 2. In addition, the binding of a protein encoded by sh06537-D to a Rho family protein was examined in a same manner, using the expression vector constructed in Example 2, which contains sh06537-D that is DNA consisting of the partial sequence and lacking a DH/PH domain coding region.

RhoA was used as a Rho family protein. An expression vector for expressing RhoA as an N-terminal GST-fusion protein was prepared using GATEWAY^{™} Cloning Technology (Invitrogen). Specifically, RhoA gene was amplified by pfu turbo, using spleen first strand DNA of Multiple Tissue cDNA Panels (Clontech) as a template. An amplified product was inserted into pENTR/D in a reaction using TOPO cloning system to prepare an entry vector. Oligonucleotides shown by the respective nucleotide sequences set forth in SEQ ID NO: 10 and SEQ ID NO: 11 were used as primers for amplification reaction. The constructed entry vector was then subjected to a recombination reaction in the presence of LR clonase, using an N-terminal GST-fusion protein-expression vector, pDEST27, to prepare a GST-fusion Rho expression plasmid Sequencing analysis confirmed correct insertion of the nucleotide sequence of a coding region of each gene. Sequencing reaction was carried out using DYEnamic ET Terminator Cycle Sequencing Kit (Amersham Biosciences). Electrophoresis and analysis were performed using ABI PRISM 377.

The sh06537-F expression vector and the RhoA gene expression vector were co-transfected into 293EBNA cells, for detecting the binding to the protein encoded by sh06537-F to a Rho family protein. Specifically, both expression vectors were added to serum-free DMEM, subsequently mixed with serum-free DMEM containing Lipofectamine^{™} 2000, and incubated at room temperature for 20 minutes. The obtained mixture was added to 293EBNA cells. The 293EBNA cells had been seeded the day before transfection at 6.0 x 10⁴ cells/well in 24 well plates, and cultured at 37 °C overnight in the presence of 5% CO₂ prior to use in this Example. The cells subjected to transfection were cultured for 2 days at 37 °C in the presence of 5% CO₂. After culturing, the cells were washed with PBS, and then lysed with lysis buffer (see Example 2 for the composition) containing 1% protease inhibitor cocktail (SIGMA) to prepare a cell lysate. On the other hand, the sh06537-D expression vector was used, instead of the sh06537-F expression vector, for transfecting 293EBNA cells together with the RhoA gene expression vector. The obtained cells were used for preparing a cell lysate in a same manner as described in Example 2. A cell lysate prepared from cells transfected only with RhoA gene expression vector was used as a negative control.

Each cell lysate was subjected to a pull-down assay for detecting the binding of a protein encoded by sh06537-F, or a protein encoded by sh06537-D, to RhoA. 300µL of each cell lysate, 20µL of Glutathione Sepharose 4B in the lysis buffer, and 100µL of the lysis buffer were mixed. Each sample was prepared in such a way that the final concentration of MgCl₂ and dithiothreitol (DTT) was 1 mM. After reacting at 4 °C for 1 hour with rotating by rotator, each sample was washed three times with 1mL of cold lysis buffer (the final concentration of MgCl₂ was 1 mM) by centrifugation at 1,000 rpm for 15 seconds at 4 °C. After washing and removing the supernatant, the Glutathione Sepharose 4B was added with 40µL of a solution that was prepared by mixing SDS-PAGE sample buffer with an equal volume of the lysis buffer. After that, it was mixed with a mixer and subjected to a heat treatment at 100 °C for 5 minutes to prepare a sample for electrophoresis. SDS-polyacrylamide gel electrophoresis was carried out, and then the gel was equilibrated for 5 minutes or more in a blotting buffer. The proteins were transferred onto a PVDF membrane from the gel. After blotting, the PVDF membrane was subjected to blocking at 4 °C overnight in TBS-T+BA. After blocking, the PVDF membrane was washed with TBS-T with shaking for 10 minutes or more. The SDS-PAGE sample buffer, the blotting buffer, TBS-T and TBS-T+BA, which were used herein, had the same components with the respective buffers used in Example 2.

Anti-FLAG M2 monoclonal antibodies (SIGMA) that was diluted to 1000-fold with TBS-T+BA was added to the PVDF membrane, and incubated at 37 °C for 1 hour or more. After that, the PVDF membrane was washed three times with TBS-T with shaking (20 minutes or more for each wash), and then HRP-labeled anti-mouse IgG antibody (Cell Signaling Technology) that was diluted to 1000-fold with TBS-T+BA was added to the membrane. The membrane was incubated at 37 °C for 1 hour or more. Finally, the PVDF membrane was washed three times with TBS-T with shaking (20 minutes or more for each wash), followed by detection of the expressed protein that reacted with anti-FLAG antibodies by using the ECL Plus Western Blotting Detection System (Amersham biosciences).
The chemiluminescence was visualized with a Lumino Imaging Analyzer (TOYOBO) detection device.

The results are shown in Figure 1. A band which indicates the binding of a protein encoded by sh06537-F to RhoA was detected in a cell lysate prepared from the cells in which the protein encoded by sh06537-F was co-expressed with RhoA (lane 2 in the upper panel of Figure 1). In contrast, a band which indicates the binding of a protein encoded by sh06537-D to RhoA was not detected in a cell lysate prepared from the cells in which the protein encoded by sh06537-D was co-expressed with RhoA (lane 3 in the upper panel of Figure 1). The expression of the protein encoded by sh06537-F or of the protein encoded by sh06537-D was observed in the cell (the middle panel of Figure 1). The protein encoded by sh06537-F and the protein encoded by sh06537-D, which were contained in the respective cell lysates, were almost the same in amount (the middle panel of Figure 1).
In addition, the amount of RhoA contained in each cell lysate was almost the same (the lower panel of Figure 1).

If the protein encoded by sh06537-F, or the protein encoded by sh06537-D, binds to RhoA, a band is detected at the position corresponding to the respective protein by using anti-FLAG antibody. The aforementioned results revealed that the protein encoded by sh06537-F binds to RhoA. The protein encoded by sh06537-D lacking a DH/PH domain coding region did not bind to RhoA. Thus, it was found that the binding of the protein encoded by sh06537-F to RhoA depends on a DH/PH domain of the protein. A DH/PH domain is an important domain participating in the Rho-GEF-mediated activation of a Rho family protein. In addition, it is considered to be an active domain of Rho-GEF. Therefore, the inventors believe that the protein encoded by sh06537-F binds to Rho family proteins such as RhoA, and thereby exhibits GEF activity.

### INDUSTRIAL APPLICABILITY

The protein, encoded by a polynucleotide according to the present invention, was bound to a Rho family protein, RhoA. The present proteins bind to a Rho family protein via a DH/PH domain. In general, Rho-GEF binds to a Rho family protein and activates the Rho family protein via a DH/PH domain. Therefore, it can be considered that the present proteins have a GEF activity for a Rho family protein. Use of the present proteins and polynucleotides allow elucidation and regulation of the Rho family protein mediated signal transduction pathway and cellular function. Further, use of the present proteins and polynucleotides allow for diagnosis, prevention and/or treatment of a disease due to an abnormal function of the present proteins and/or an abnormal expression of the present polynucleotides, for example, a tumor disease such as tonsil tumors and/or bronchial tumors. Thus, the present invention is extremely useful in a wide field including basic research and pharmaceutical development.

### GENERAL DESCRIPTION OF THE SEQUENCES

SEQ ID NO: 1: polynucleotide that encodes the protein (SEQ ID NO: 2) having a function as a guanine nucleotide exchange factor.
SEQ ID NO: 1: (3706): (4245) a region encoding a Db1 homology domain.
SEQ ID NO: 1: (4345): (4680) a region encoding a pleckstrin homology domain.
SEQ ID NO: 3: partial sequence of SEQ ID NO: 1 consisting of the nucleotides from the 175^{th} to the 3693^{rd}, which lacks a region encoding a Db1 homology domain and a pleckstrin homology domain.
SEQ ID NO: 3: polynucleotide encoding the amino acid sequence set forth in SEQ ID NO: 4.
SEQ ID NO: 5: designed oligonucleotide based on the sequence of SEQ ID NO: 1 for use as a primer.
SEQ ID NO: 6: designed oligonucleotide based on the sequence of SEQ ID NO: 1 for use as a primer.
SEQ ID NO: 7: designed oligonucleotide based on the sequence of SEQ ID NO: 1 for use as a primer.
SEQ ID NO: 8: polynucleotide encoding RhoA.
SEQ ID NO:9:RhoA.
SEQ ID NO: 10: designed oligonucleotide based on the sequence of SEQ ID NO: 8 for use as a primer.
SEQ ID NO: 11: designed oligonucleotide based on the sequence of SEQ ID NO: 8 for use as a primer.
SEQ ID NO: 12: designed oligonucleotide for use as a primer.

## Claims

1. A polynucleotide shown by the nucleotide sequence set forth in SEQ ID NO: 1 in the sequence listing or by the complementary nucleotide sequence, or a polynucleotide encoding a protein shown by the amino acid sequence set forth in SEQ ID NO: 2 in the sequence listing, or a polynucleotide shown by the complementary nucleotide sequence of the polynucleotide.

2. A polynucleotide selected from the following group, wherein the polynucleotide encodes a protein that binds to RhoA:
(i) a polynucleotide shown by a nucleotide sequence having a homology of at least 70% with the nucleotide sequence of the aforementioned polynucleotide,
(ii) a polynucleotide with a mutation or an induced mutation, such as deletion, substitution, addition of one or several nucleotides in the nucleotide sequence of the aforementioned polynucleotide, and
(iii) a polynucleotide that hybridizes to the aforementioned polynucleotide under stringent conditions.

3. A polynucleotide selected from the following group, wherein the polynucleotide encodes a protein having a guanine nucleotide exchange factor (GEF) activity for RhoA:
(i) a polynucleotide shown by a nucleotide sequence having a homology of at least 70% with the nucleotide sequence of the aforementioned polynucleotide,
(ii) a polynucleotide with a mutation or an induced mutation, such as deletion, substitution, addition of one or several nucleotides in the nucleotide sequence of the aforementioned polynucleotide, and
(iii) a polynucleotide that hybridizes to the aforementioned polynucleotide under stringent conditions.

4. A recombinant vector containing the polynucleotide according to any one of claims 1 to 3.

5. A transformant that has been transfected with the recombinant vector according to claim 4.

6. The transformant according to claim 5, which has been transfected with the recombinant vector according to claim 4, and a recombinant vector containing a polynucleotide encoding RhoA.

7. A protein shown by the amino acid sequence set forth in SEQ ID NO: 2, in the sequence listing.

8. A protein encoded by the polynucleotide according to claim 2 or claim 3.

9. A method of producing the protein according to claim 7 or claim 8, comprising culturing the transformant according to claim 5 or claim 6.

10. An antibody that recognizes the protein according to claim 7 or claim 8.

11. A method of identifying a compound that inhibits the function of the proteins according to claim 7 or claim 8, and/or the expression of the polynucleotides according to any one of claims 1 to 3, comprising detecting the presence, absence or change in the function and/or the expression under conditions where the interaction of a compound with the protein and/or the polynucleotide are allowed, and determining whether the compound inhibits the function of the protein and/or the expression of the polynucleotide.

12. The method according to claim 11, wherein the function of the protein is a guanine nucleotide exchange factor (GEF) activity for RhoA.

13. A method of determining whether a tissue specimen derived from a human tonsil tissue is a tissue derived from a human tonsil tumor or not, comprising measuring an amount of expression of the polynucleotide according to any one of claims 1 to 3 in the tissue specimen.

14. The method according to claim 13, wherein the method determines that the tissue specimen is a tissue derived from a human tonsil tumor in the case when the amount of expression of the polynucleotide according to any one of claims 1 to 3 in the tissue specimen is 2.5 times higher than that in a control tissue derived from normal human tonsil.

15. A method of determining whether a tissue specimen derived from a human bronchial tissue is a tissue derived from a human bronchial tumor or not, comprising measuring an amount of expression of the polynucleotide according to any one of claims 1 to 3 in the tissue specimen.

16. The method according to claim 15, wherein the method determines that the tissue specimen is a tissue derived from a human bronchial tumor in the case when the amount of expression of the polynucleotide according to any one of claims 1 to 3 in the tissue specimen is 1.5 times higher than that in a control tissue derived from normal human bronchia.

17. An agent for preventing and/or treating tonsil tumors and/or bronchial tumors, comprising a compound that inhibits the function of the protein according to claim 7 or claim 8 and/or a compound that inhibits the expression of the polynucleotide according to any one of claims 1 to 3, as an active ingredient.

18. A method of preventing and/or treating tonsil tumors and/or bronchial tumors, comprising using a compound that inhibits the function of the protein according to claim 7 or claim 8 and/or a compound that inhibits the expression of the polynucleotide according to any one of claims 1 to 3.

19. A reagent kit containing at least one selected from the protein according to claim 7 or claim 8, the polynucleotide according to any one of claims 1 to 3, the recombinant vector according to claim 4, the transformant according to claim 5 or claim 6, and the antibody according to claim 10.


SEQUENCE LISTING

<110>  KAZUSA DNA RESEARCH INSTITUTE FOUNDATION
       DAIICHI PHARMACEUTICAL CO., LTD.

<120>  A gene encoding a guanine nucleotide exchange factor that can bind to RhoA

<130>  DAII-004-EP

<140>  EP 05734386.5
       2005-04-20

<150>  JP P2004-125997
<151>  2004-04-21

<160>  12

<170>  PatentIn version 3.1

<210>  1
<211>  5541
<212>  DNA
<213>  homo sapiens

<220>
<221>  misc_feature
<223>  A polynucleotide encoding the protein (SEQ ID NO:2) that have a f
       unction of guanine nucleotide exchange factor.


<220>
<221>  CDS
<222>  (175)..(4968)
<223>

<220>
<221>  misc_feature
<222>  (3706)..(4245)
<223>  A region encoding Dbl homology domain.


<220>
<221>  misc_feature
<222>  (4345)..(4680)
<223>  A region encoding Pleckstrin homology domain.


<400>  1
   gaaacttcct aggagaaccc gatccctccc tcccgtcggg cggccagggg cgggccgcgg      60
   gtggggcggc cgggcctgcg ctggggacgg ctctggggac tgcggccggc gccgggacct     120
   ggaggggacg ctggggccga agcagcatgt gacaccgacc aggattcagc cctg atg       177
                                                               Met
                                                               1
   gag gct gag gag gcc cag cgt gga gcc tct cct ccc atc tct gcc ata       225
   Glu Ala Glu Glu Ala Gln Arg Gly Ala Ser Pro Pro Ile Ser Ala Ile
               5                   10                  15
   gag gaa ttc agc att atc cct gag gct ccc atg agg agc agc cag gtc       273
   Glu Glu Phe Ser Ile Ile Pro Glu Ala Pro Met Arg Ser Ser Gln Val
           20                  25                  30
   tct gcc ttg ggg ctt gaa gcc caa gaa gat gag gac cca tcc tat aag       321
   Ser Ala Leu Gly Leu Glu Ala Gln Glu Asp Glu Asp Pro Ser Tyr Lys
       35                  40                  45
   tgg aga gag gaa cac aga ctc tca gca act cag cag agt gag tta agg       369
   Trp Arg Glu Glu His Arg Leu Ser Ala Thr Gln Gln Ser Glu Leu Arg
   50                  55                  60                  65
   gat gtg tgt gac tat gcg att gag acg atg ccc tct ttt ccc aag gaa       417
   Asp Val Cys Asp Tyr Ala Ile Glu Thr Met Pro Ser Phe Pro Lys Glu
                   70                  75                  80
   ggt tct gca gat gtg gag ccc aat cag gaa agc ctt gtg gct gag gcc 465
   Gly Ser Ala Asp Val Glu Pro Asn Gln Glu Ser Leu Val Ala Glu Ala
   85 90 95
   tgt gac act ccg gaa cac tgg gag gca gta ccc cag agc cta gca ggc 513
   Cys Asp Thr Pro Glu His Trp Glu Ala Val Pro Gln Ser Leu Ala Gly
   100 105 110
   cga caa gca agg act cta gct ccc cca gag ctc tgg gcc tgc ccc att 561
   Arg Gln Ala Arg Thr Leu Ala Pro Pro Glu Leu Trp Ala Cys Pro Ile
   115 120 125
   cag agt gag cat cta gac atg gcc cca ttt tcc agt gac ctg gga agc 609
   Gln Ser Glu His Leu Asp Met Ala Pro Phe Ser Ser Asp Leu Gly Ser
   130 135 140 145
   gaa gaa gag gag gtg gaa ttt tgg cca gga ctt act tct ttg aca ttg 657
   Glu Glu Glu Glu Val Glu Phe Trp Pro Gly Leu Thr Ser Leu Thr Leu
   150 155 160
   gga tct gga cag gca gaa gaa gaa gag gaa acc tct tca gat aac tct 705
   Gly Ser Gly Gln Ala Glu Glu Glu Glu Glu Thr Ser Ser Asp Asn Ser
   165 170 175
   ggt cag acc aga tat tat tct ccc tgc gaa gag cat cct gca gag acc 753
   Gly Gln Thr Arg Tyr Tyr Ser Pro Cys Glu Glu His Pro Ala Glu Thr
   180 185 190
   aac cag aat gaa ggc tct gaa agt ggg act atc agg cag ggg gaa gag 801
   Asn Gln Asn Glu Gly Ser Glu Ser Gly Thr Ile Arg Gln Gly Glu Glu
   195 200 205
   ctg cca cct gag gag ctg cag gaa agt caa ggg ctc ttg cat ccc cag 849
   Leu Pro Pro Glu Glu Leu Gln Glu Ser Gln Gly Leu Leu His Pro Gln
   210 215 220 225
   gag gtc caa gtt ctg gag gag cag gga cag cag gaa gca gga ttt cgg 897
   Glu Val Gln Val Leu Glu Glu Gln Gly Gln Gln Glu Ala Gly Phe Arg
   230 235 240
   ggg gaa gga act ctg agg gag gat gtt tgt gcc gat ggg cta tta ggg 945
   Gly Glu Gly Thr Leu Arg Glu Asp Val Cys Ala Asp Gly Leu Leu Gly
   245 250 255
   gag gaa cag atg ata gag cag gtt aat gat gaa aag gga gaa cag aag 993
   Glu Glu Gln Met Ile Glu Gln Val Asn Asp Glu Lys Gly Glu Gln Lys
   260 265 270
   caa aaa cag gaa cag gta caa gat gtg atg ctt ggg aga caa gga gaa 1041
   Gln Lys Gln Glu Gln Val Gln Asp Val Met Leu Gly Arg Gln Gly Glu
   275 280 285
   aga atg ggg ctc act ggg gag cca gag ggt ctg aat gac ggt gag tgg 1089
   Arg Met Gly Leu Thr Gly Glu Pro Glu Gly Leu Asn Asp Gly Glu Trp
   290 295 300 305
   gag cag gag gat atg gag agg aag gct cag ggt cag gga ggt cca gaa 1137
   Glu Gln Glu Asp Met Glu Arg Lys Ala Gln Gly Gln Gly Gly Pro Glu
   310 315 320
   cag gga gaa gag agg aag agg gag ctg cag gtg cca gaa gag aat agg 1185
   Gln Gly Glu Glu Arg Lys Arg Glu Leu Gln Val Pro Glu Glu Asn Arg
   325 330 335
   gcg gac tct cag gac gaa aag agt caa acc ttt ttg gga aaa tca gag 1233
   Ala Asp Ser Gln Asp Glu Lys Ser Gln Thr Phe Leu Gly Lys Ser Glu
   340 345 350
   gaa gta act gga aag caa gaa gat cat ggt ata aag gag aaa ggg gtg 1281
   Glu Val Thr Gly Lys Gln Glu Asp His Gly Ile Lys Glu Lys Gly Val
   355 360 365
   cca gtc agc ggg cag gag gcg aaa gag cca gag agt tgg gat ggg ggc 1329
   Pro Val Ser Gly Gln Glu Ala Lys Glu Pro Glu Ser Trp Asp Gly Gly
   370 375 380 385
   agg ctg ggg gca gtg gga aga gcg agg agc agg gaa gag gag aat gag 1377
   Arg Leu Gly Ala Val Gly Arg Ala Arg Ser Arg Glu Glu Glu Asn Glu
   390 395 400
   cat cat ggg cct tca atg ccc gct ctg ata gcc cct gag gac tct cct 1425
   His His Gly Pro Ser Met Pro Ala Leu Ile Ala Pro Glu Asp Ser Pro
   405 410 415
   cac tgt gac ctg ttt cca ggt gcc tca tat ctc atg act cag att ccc 1473
   His Cys Asp Leu Phe Pro Gly Ala Ser Tyr Leu Met Thr Gln Ile Pro
   420 425 430
   ggg act cag aca gag tcc agg gct gag gaa ctg tcc ccc gca gct ctg 1521
   Gly Thr Gln Thr Glu Ser Arg Ala Glu Glu Leu Ser Pro Ala Ala Leu
   435 440 445
   tct ccc tcg tta gag ccc atc agg tgc tct cac cag ccc att tct cta 1569
   Ser Pro Ser Leu Glu Pro Ile Arg Cys Ser His Gln Pro Ile Ser Leu
   450 455 460 465
   ctg ggc tcc ttt ttg act gag gag tca cct gac aag gaa ata gat caa 1617
   Leu Gly Ser Phe Leu Thr Glu Glu Ser Pro Asp Lys Glu Ile Asp Gln
   470 475 480
   aac agc cag caa gag gga tcc agg ctg agg aag gga aca gtg tcc agc 1665
   Asn Ser Gln Gln Glu Gly Ser Arg Leu Arg Lys Gly Thr Val Ser Ser
   485 490 495
   caa ggg act gag gtg gtc ttt gcc agt gca tct gtg act cct cca agg 1713
   Gln Gly Thr Glu Val Val Phe Ala Ser Ala Ser Val Thr Pro Pro Arg
   500 505 510
   aca cca gat tca gct cct ccc agt cct gct gaa gcc tac ccc atc aca 1761
   Thr Pro Asp Ser Ala Pro Pro Ser Pro Ala Glu Ala Tyr Pro Ile Thr
   515 520 525
   cct gcc tcg gta tct gcc agg ccc cca gtt gcc ttt ccc agg agg gaa 1809
   Pro Ala Ser Val Ser Ala Arg Pro Pro Val Ala Phe Pro Arg Arg Glu
   530 535 540 545
   acc tct tgt gct gca cgt gct cca gaa act gcc agt gcc cct ctc tca 1857
   Thr Ser Cys Ala Ala Arg Ala Pro Glu Thr Ala Ser Ala Pro Leu Ser
   550 555 560
   atg gat gac cca tct ccc tgt ggg act tct gag atg tgc cag gct gcc 1905
   Met Asp Asp Pro Ser Pro Cys Gly Thr Ser Glu Met Cys Gln Ala Ala
   565 570 575
   ctc tat ggc ttc ccc tcc acc ggg acc agc cct cca agg ccc cca gcc 1953
   Leu Tyr Gly Phe Pro Ser Thr Gly Thr Ser Pro Pro Arg Pro Pro Ala
   580 585 590
   aac tcc aca ggc acc gtc cag cac tta cgg agt gac tcc ttc cct ggt 2001
   Asn Ser Thr Gly Thr Val Gln His Leu Arg Ser Asp Ser Phe Pro Gly
   595 600 605
   tct cac agg aca gag cag act cca gac ctg gtg gga atg ttg ctt tcc 2049
   Ser His Arg Thr Glu Gln Thr Pro Asp Leu Val Gly Met Leu Leu Ser
   610 615 620 625
   tac tcc cac tca gag ctg ccc cag agg ccc ccc aaa cct gcc atc tac 2097
   Tyr Ser His Ser Glu Leu Pro Gln Arg Pro Pro Lys Pro Ala Ile Tyr
   630 635 640
   agc tct gtg acc cca aga agg gac aga agg agt ggt agg gac tac agc 2145
   Ser Ser Val Thr Pro Arg Arg Asp Arg Arg Ser Gly Arg Asp Tyr Ser
   645 650 655
   acc gtt tca gca tcc cct act gcc tta tcc acg ctg aag cag gac tct 2193
   Thr Val Ser Ala Ser Pro Thr Ala Leu Ser Thr Leu Lys Gln Asp Ser
   660 665 670
   caa gaa tcc atc tca aat cta gag aga ccc agc agt cct ccc agc atc 2241
   Gln Glu Ser Ile Ser Asn Leu Glu Arg Pro Ser Ser Pro Pro Ser Ile
   675 680 685
   cag ccc tgg gtc tcc cca cat aat cca gcc ttt gcc aca gag tct ccc 2289
   Gln Pro Trp Val Ser Pro His Asn Pro Ala Phe Ala Thr Glu Ser Pro
   690 695 700 705
   gcc tac ggt tct tcc cca tcc ttt gtc tcc atg gag gat gtg agg atc 2337
   Ala Tyr Gly Ser Ser Pro Ser Phe Val Ser Met Glu Asp Val Arg Ile
   710 715 720
   cac gaa cct ctg ccc cct cct ccc cca cag agg agg gac acc cat ccc 2385
   His Glu Pro Leu Pro Pro Pro Pro Pro Gln Arg Arg Asp Thr His Pro
   725 730 735
   tcc gtg gtg gag aca gat ggc cat gct cgt gta gtg gtt ccc acg ctg 2433
   Ser Val Val Glu Thr Asp Gly His Ala Arg Val Val Val Pro Thr Leu
   740 745 750
   aag cag cat agc cac cct cct cca ttg gcc cta ggt tca ggg ctg cat 2481
   Lys Gln His Ser His Pro Pro Pro Leu Ala Leu Gly Ser Gly Leu His
   755 760 765
   gcc ccc cat aaa ggc cca ctt ccc caa gcc tct gac ccc gct gtg gcc 2529
   Ala Pro His Lys Gly Pro Leu Pro Gln Ala Ser Asp Pro Ala Val Ala
   770 775 780 785
   agg cag cac cga cct ctg cca tct acc cca gac agc tcc cac cat gct 2577
   Arg Gln His Arg Pro Leu Pro Ser Thr Pro Asp Ser Ser His His Ala
   790 795 800
   cag gcc acc ccc agg tgg aga tac aac aag ccg cta ccc cct acc cct 2625
   Gln Ala Thr Pro Arg Trp Arg Tyr Asn Lys Pro Leu Pro Pro Thr Pro
   805 810 815
   gat ttg ccg cag ccc cac ctt cct ccc att tct gct cct ggt agc tca 2673
   Asp Leu Pro Gln Pro His Leu Pro Pro Ile Ser Ala Pro Gly Ser Ser
   820 825 830
   agg atc tac agg cct cta ccc cca cta ccc atc ata gac cct ccc acc 2721
   Arg Ile Tyr Arg Pro Leu Pro Pro Leu Pro Ile Ile Asp Pro Pro Thr
   835 840 845
   gaa cca ccc cca ttg ccc cca aag tcc agg ggg agg agc agg agc act 2769
   Glu Pro Pro Pro Leu Pro Pro Lys Ser Arg Gly Arg Ser Arg Ser Thr
   850 855 860 865
   cgg gga gga cat atg aac tca ggg ggt cat gcc aaa aca aga cct gct 2817
   Arg Gly Gly His Met Asn Ser Gly Gly His Ala Lys Thr Arg Pro Ala
   870 875 880
   tgt caa gac tgg aca gtc ccc ctc cct gcc tct gct gga cgc acc tcc 2865
   Cys Gln Asp Trp Thr Val Pro Leu Pro Ala Ser Ala Gly Arg Thr Ser
   885 890 895
   tgg ccc ccg gcc aca gct aga tca aca gag tct ttc act tcc acc agc 2913
   Trp Pro Pro Ala Thr Ala Arg Ser Thr Glu Ser Phe Thr Ser Thr Ser
   900 905 910
   agg agt aag agc gaa gtg tcc cct ggc atg gct ttc agc aac atg aca 2961
   Arg Ser Lys Ser Glu Val Ser Pro Gly Met Ala Phe Ser Asn Met Thr
   915 920 925
   aac ttc cta tgc ccc tct tcc cct acc act ccc tgg act ccg gag ctc 3009
   Asn Phe Leu Cys Pro Ser Ser Pro Thr Thr Pro Trp Thr Pro Glu Leu
   930 935 940 945
   cag gga ccc acc tct aag gat gaa gca ggg gtc tca gaa cac cct gag 3057
   Gln Gly Pro Thr Ser Lys Asp Glu Ala Gly Val Ser Glu His Pro Glu
   950 955 960
   gcc cct gcg aga gaa cct ttg aga agg aca acc cct cag caa gga gcc 3105
   Ala Pro Ala Arg Glu Pro Leu Arg Arg Thr Thr Pro Gln Gln Gly Ala
   965 970 975
   agt ggc cca ggg agg tca cct gtg ggc caa gca agg cag cca gaa aaa 3153
   Ser Gly Pro Gly Arg Ser Pro Val Gly Gln Ala Arg Gln Pro Glu Lys
   980 985 990
   ccc agc cat ctg cac ctg gag aag gcg tcc agc tgg ccc cac agg cgg 3201
   Pro Ser His Leu His Leu Glu Lys Ala Ser Ser Trp Pro His Arg Arg
   995 1000 1005
   gac tca ggg agg cca cca ggg gac agc agt gga cag gct gtg gct 3246
   Asp Ser Gly Arg Pro Pro Gly Asp Ser Ser Gly Gln Ala Val Ala
   1010 1015 1020
   cct agt gag ggg gcc aac aag cac aag ggc tgg agc cgg cag ggc 3291
   Pro Ser Glu Gly Ala Asn Lys His Lys Gly Trp Ser Arg Gln Gly
   1025 1030 1035
   ctg cgc aga cct tcc atc ttg cct gag ggc tct tca gat tca aga 3336
   Leu Arg Arg Pro Ser Ile Leu Pro Glu Gly Ser Ser Asp Ser Arg
   1040 1045 1050
   ggt cca gcc gtg gag aaa cat ccg gga ccc tca gac act gtt gtt 3381
   Gly Pro Ala Val Glu Lys His Pro Gly Pro Ser Asp Thr Val Val
   1055 1060 1065
   ttt cgg gag aaa aaa cca aag gag gtg atg gga ggc ttt tca aga 3426
   Phe Arg Glu Lys Lys Pro Lys Glu Val Met Gly Gly Phe Ser Arg
   1070 1075 1080
   cgc tgc tcc aaa ctc atc aac tcc tcc cag ctg ctt tac cag gag 3471
   Arg Cys Ser Lys Leu Ile Asn Ser Ser Gln Leu Leu Tyr Gln Glu
   1085 1090 1095
   tat agt gat gtt gtc ctg aat aag gag atc cag agc cag cag cgg 3516
   Tyr Ser Asp Val Val Leu Asn Lys Glu Ile Gln Ser Gln Gln Arg
   1100 1105 1110
   ctg gag agc ctg tcc gag aca ccc ggg cct agc tct ccg cgg cag 3561
   Leu Glu Ser Leu Ser Glu Thr Pro Gly Pro Ser Ser Pro Arg Gln
   1115 1120 1125
   cct cgg aag gcc ctg gtc tcc tcc gag tcg tac ctg cag cgg ctc 3606
   Pro Arg Lys Ala Leu Val Ser Ser Glu Ser Tyr Leu Gln Arg Leu
   1130 1135 1140
   tcc atg gcc tcc agc ggc tcc ctc tgg cag gaa atc ccc gtg gtg 3651
   Ser Met Ala Ser Ser Gly Ser Leu Trp Gln Glu Ile Pro Val Val
   1145 1150 1155
   cgc aac agc acc gtg ctg ctc tcc atg acc cat gaa gac caa aag 3696
   Arg Asn Ser Thr Val Leu Leu Ser Met Thr His Glu Asp Gln Lys
   1160 1165 1170
   ctg caa gag gtc aaa ttt gag ctg att gtg tca gag gcc tcc tac 3741
   Leu Gln Glu Val Lys Phe Glu Leu Ile Val Ser Glu Ala Ser Tyr
   1175 1180 1185
   ctg cgc agt cta aac ata gct gtg gat cat ttc caa ctt tca act 3786
   Leu Arg Ser Leu Asn Ile Ala Val Asp His Phe Gln Leu Ser Thr
   1190 1195 1200
   tca ctc cgg gcc aca ctt tcc aac cag gag cac caa tgg ctc ttc 3831
   Ser Leu Arg Ala Thr Leu Ser Asn Gln Glu His Gln Trp Leu Phe
   1205 1210 1215
   tct cgt tta cag gat gtg cga gac gtc agc gcc acg ttc ctt tca 3876
   Ser Arg Leu Gln Asp Val Arg Asp Val Ser Ala Thr Phe Leu Ser
   1220 1225 1230
   gac ctg gaa gag aac ttt gag aac aat atc ttc tcc ttc caa gta 3921
   Asp Leu Glu Glu Asn Phe Glu Asn Asn Ile Phe Ser Phe Gln Val
   1235 1240 1245
   tgt gac gta gtc ctg aac cac gcc cca gac ttc cgc cgg gtc tac 3966
   Cys Asp Val Val Leu Asn His Ala Pro Asp Phe Arg Arg Val Tyr
   1250 1255 1260
   ctg cct tat gtc acc aac cag acc tat cag gaa cgc acc ttc cag 4011
   Leu Pro Tyr Val Thr Asn Gln Thr Tyr Gln Glu Arg Thr Phe Gln
   1265 1270 1275
   agc ctg atg aat agc aac agc aat ttc cgg gag gtc ttg gag aag 4056
   Ser Leu Met Asn Ser Asn Ser Asn Phe Arg Glu Val Leu Glu Lys
   1280 1285 1290
   ctg gag agc gac ccc gtc tgc cag cgc ctt tcc ctc aag tcc ttt 4101
   Leu Glu Ser Asp Pro Val Cys Gln Arg Leu Ser Leu Lys Ser Phe
   1295 1300 1305
   ctg att ctg ccc ttc caa cgc atc acc cgc ctc aaa ctg ctg ctc 4146
   Leu Ile Leu Pro Phe Gln Arg Ile Thr Arg Leu Lys Leu Leu Leu
   1310 1315 1320
   cag aac att ctg aag aga aca cag cct ggc tcc tcg gag gag gca 4191
   Gln Asn Ile Leu Lys Arg Thr Gln Pro Gly Ser Ser Glu Glu Ala
   1325 1330 1335
   gag gcc acg aag gca cac cac gcc ctg gag cag ctg atc cgg gac 4236
   Glu Ala Thr Lys Ala His His Ala Leu Glu Gln Leu Ile Arg Asp
   1340 1345 1350
   tgc aat aac aat gtc cag agt atg cga cgg aca gag gag cta atc 4281
   Cys Asn Asn Asn Val Gln Ser Met Arg Arg Thr Glu Glu Leu Ile
   1355 1360 1365
   tac ctg agc cag aag att gag ttt gag tgc aaa ata ttc ccg ctc 4326
   Tyr Leu Ser Gln Lys Ile Glu Phe Glu Cys Lys Ile Phe Pro Leu
   1370 1375 1380
   att tct cag tca cgc tgg ctg gtg aaa agt ggg gag ctg aca gcc 4371
   Ile Ser Gln Ser Arg Trp Leu Val Lys Ser Gly Glu Leu Thr Ala
   1385 1390 1395
   ttg gag ttc agt gct tcc cca ggg cta cga agg aag ctg aac acg 4416
   Leu Glu Phe Ser Ala Ser Pro Gly Leu Arg Arg Lys Leu Asn Thr
   1400 1405 1410
   cgt cca gtc cac ctg cac ctc ttc aat gac tgt ctg ctg ctg tct 4461
   Arg Pro Val His Leu His Leu Phe Asn Asp Cys Leu Leu Leu Ser
   1415 1420 1425
   cgg ccc cga gag ggt agc cga ttc ctg gta ttt gac cat gct ccc 4506
   Arg Pro Arg Glu Gly Ser Arg Phe Leu Val Phe Asp His Ala Pro
   1430 1435 1440
   ttc tcc tcc att cgg ggg gaa aag tgt gaa atg aag cta cat gga 4551
   Phe Ser Ser Ile Arg Gly Glu Lys Cys Glu Met Lys Leu His Gly
   1445 1450 1455
   cct cac aaa aac ctg ttc cga ctc ttt ctg cgg cag aac act cag 4596
   Pro His Lys Asn Leu Phe Arg Leu Phe Leu Arg Gln Asn Thr Gln
   1460 1465 1470
   ggc gcc cag gcc gag ttc ctc ttc cgc acg gag act caa agt gaa 4641
   Gly Ala Gln Ala Glu Phe Leu Phe Arg Thr Glu Thr Gln Ser Glu
   1475 1480 1485
   aag ctt cgg tgg atc tca gcc ttg gcc atg cca aga gag gag ttg 4686
   Lys Leu Arg Trp Ile Ser Ala Leu Ala Met Pro Arg Glu Glu Leu
   1490 1495 1500
   gac ctt ctg gag tgt tac aac tcc ccc cag gta cag tgc ctt cga 4731
   Asp Leu Leu Glu Cys Tyr Asn Ser Pro Gln Val Gln Cys Leu Arg
   1505 1510 1515
   gcc tac aag ccc cga gag aat gat gaa ttg gca ctg gag aaa gcc 4776
   Ala Tyr Lys Pro Arg Glu Asn Asp Glu Leu Ala Leu Glu Lys Ala
   1520 1525 1530
   gac gtg gtg atg gtg act cag cag agc agt gac ggc tgg ctg gag 4821
   Asp Val Val Met Val Thr Gln Gln Ser Ser Asp Gly Trp Leu Glu
   1535 1540 1545
   ggc gtg agg ctc tca gac ggg gag cga ggc tgg ttt cct gtg cag 4866
   Gly Val Arg Leu Ser Asp Gly Glu Arg Gly Trp Phe Pro Val Gln
   1550 1555 1560
   cag gtg gag ttc att tcc aac cca gag gtc cgt gca cag aac ctg 4911
   Gln Val Glu Phe Ile Ser Asn Pro Glu Val Arg Ala Gln Asn Leu
   1565 1570 1575
   aag gaa gct cat cga gtc aag act gcc aaa cta cag ctg gtg gaa 4956
   Lys Glu Ala His Arg Val Lys Thr Ala Lys Leu Gln Leu Val Glu
   1580 1585 1590
   cag caa gcc taa gtcttctctg agaggagttt cgtgagctga agaacaagct 5008
   Gln Gln Ala
   1595
   gctcatggca agggctggcc ccagaaccct gcaagagagg ccttctgtgg atggagaact 5068
   aggccttctc aaagctcaag gacaaaatcc agctaaccca gtccctcggc ccaggcctcc 5128
   tttcgtgctt tgtgcttggt gggggggatt tcgagggact ttgcactgga ctctgggaac 5188
   ctttcatcat taaaaaaagg gggaccattg gggcctgagc caaggaactt tccttctact 5248
   gccttatagt gcttaaacat tctccgcctc cagggtgcag attcagagct ggccagagtt 5308
   tcagtgatag ccgtatgtta aacagaatct cacctcagtc tcctggaggg agatgtttaa 5368
   gaggggttaa cacatcagat gggagggtca gcccggtgac ctctaaggta tcttctaacc 5428
   tagaaactca ccataattat ggtgccaagg tcagtgtgtc tctgagatct atgtctgttg 5488
   gtggcaatgt gagggtgata ctctctcact ctaataaact tggcacttct ccg 5541

<210> 2
<211> 1597
<212> PRT
<213> homo sapiens

<400> 2

Met Glu Ala Glu Glu Ala Gln Arg Gly Ala Ser Pro Pro Ile Ser Ala
1               5                   10                  15

Ile Glu Glu Phe Ser Ile Ile Pro Glu Ala Pro Met Arg Ser Ser Gln
            20                  25                  30

Val Ser Ala Leu Gly Leu Glu Ala Gln Glu Asp Glu Asp Pro Ser Tyr
        35                  40                  45

Lys Trp Arg Glu Glu His Arg Leu Ser Ala Thr Gln Gln Ser Glu Leu
    50                  55                  60

Arg Asp Val Cys Asp Tyr Ala Ile Glu Thr Met Pro Ser Phe Pro Lys
65                  70                  75                  80

Glu Gly Ser Ala Asp Val Glu Pro Asn Gln Glu Ser Leu Val Ala Glu
                85                  90                  95

Ala Cys Asp Thr Pro Glu His Trp Glu Ala Val Pro Gln Ser Leu Ala
            100                 105                 110

Gly Arg Gln Ala Arg Thr Leu Ala Pro Pro Glu Leu Trp Ala Cys Pro
        115                 120                 125

Ile Gln Ser Glu His Leu Asp Met Ala Pro Phe Ser Ser Asp Leu Gly
    130                 135                 140

Ser Glu Glu Glu Glu Val Glu Phe Trp Pro Gly Leu Thr Ser Leu Thr
145                 150                 155                 160

Leu Gly Ser Gly Gln Ala Glu Glu Glu Glu Glu Thr Ser Ser Asp Asn
                165                 170                 175

Ser Gly Gln Thr Arg Tyr Tyr Ser Pro Cys Glu Glu His Pro Ala Glu
            180                 185                 190

Thr Asn Gln Asn Glu Gly Ser Glu Ser Gly Thr Ile Arg Gln Gly Glu
        195                 200                 205

Glu Leu Pro Pro Glu Glu Leu Gln Glu Ser Gln Gly Leu Leu His Pro
    210                 215                 220

Gln Glu Val Gln Val Leu Glu Glu Gln Gly Gln Gln Glu Ala Gly Phe
225                 230                 235                 240

Arg Gly Glu Gly Thr Leu Arg Glu Asp Val Cys Ala Asp Gly Leu Leu
                245                 250                 255

Gly Glu Glu Gln Met Ile Glu Gln Val Asn Asp Glu Lys Gly Glu Gln
            260                 265                 270

Lys Gln Lys Gln Glu Gln Val Gln Asp Val Met Leu Gly Arg Gln Gly
        275                 280                 285

Glu Arg Met Gly Leu Thr Gly Glu Pro Glu Gly Leu Asn Asp Gly Glu
    290                 295                 300

Trp Glu Gln Glu Asp Met Glu Arg Lys Ala Gln Gly Gln Gly Gly Pro
305                 310                 315                 320

Glu Gln Gly Glu Glu Arg Lys Arg Glu Leu Gln Val Pro Glu Glu Asn
                325                 330                 335

Arg Ala Asp Ser Gln Asp Glu Lys Ser Gln Thr Phe Leu Gly Lys Ser
            340                 345                 350

Glu Glu Val Thr Gly Lys Gln Glu Asp His Gly Ile Lys Glu Lys Gly
        355                 360                 365

Val Pro Val Ser Gly Gln Glu Ala Lys Glu Pro Glu Ser Trp Asp Gly
    370                 375                 380

Gly Arg Leu Gly Ala Val Gly Arg Ala Arg Ser Arg Glu Glu Glu Asn
385                 390                 395                 400

Glu His His Gly Pro Ser Met Pro Ala Leu Ile Ala Pro Glu Asp Ser
                405                 410                 415

Pro His Cys Asp Leu Phe Pro Gly Ala Ser Tyr Leu Met Thr Gln Ile
            420                 425                 430

Pro Gly Thr Gln Thr Glu Ser Arg Ala Glu Glu Leu Ser Pro Ala Ala
        435                 440                 445

Leu Ser Pro Ser Leu Glu Pro Ile Arg Cys Ser His Gln Pro Ile Ser
    450                 455                 460

Leu Leu Gly Ser Phe Leu Thr Glu Glu Ser Pro Asp Lys Glu Ile Asp
465                 470                 475                 480

Gln Asn Ser Gln Gln Glu Gly Ser Arg Leu Arg Lys Gly Thr Val Ser
                485                 490                 495

Ser Gln Gly Thr Glu Val Val Phe Ala Ser Ala Ser Val Thr Pro Pro
            500                 505                 510

Arg Thr Pro Asp Ser Ala Pro Pro Ser Pro Ala Glu Ala Tyr Pro Ile
        515                 520                 525

Thr Pro Ala Ser Val Ser Ala Arg Pro Pro Val Ala Phe Pro Arg Arg
    530                 535                 540

Glu Thr Ser Cys Ala Ala Arg Ala Pro Glu Thr Ala Ser Ala Pro Leu
545                 550                 555                 560

Ser Met Asp Asp Pro Ser Pro Cys Gly Thr Ser Glu Met Cys Gln Ala
                565                 570                 575

Ala Leu Tyr Gly Phe Pro Ser Thr Gly Thr Ser Pro Pro Arg Pro Pro
            580                 585                 590

Ala Asn Ser Thr Gly Thr Val Gln His Leu Arg Ser Asp Ser Phe Pro
        595                 600                 605

Gly Ser His Arg Thr Glu Gln Thr Pro Asp Leu Val Gly Met Leu Leu
    610                 615                 620

Ser Tyr Ser His Ser Glu Leu Pro Gln Arg Pro Pro Lys Pro Ala Ile
625                 630                 635                 640

Tyr Ser Ser Val Thr Pro Arg Arg Asp Arg Arg Ser Gly Arg Asp Tyr
                645                 650                 655

Ser Thr Val Ser Ala Ser Pro Thr Ala Leu Ser Thr Leu Lys Gln Asp
            660                 665                 670

Ser Gln Glu Ser Ile Ser Asn Leu Glu Arg Pro Ser Ser Pro Pro Ser
        675                 680                 685

Ile Gln Pro Trp Val Ser Pro His Asn Pro Ala Phe Ala Thr Glu Ser
    690                 695                 700

Pro Ala Tyr Gly Ser Ser Pro Ser Phe Val Ser Met Glu Asp Val Arg
705                 710                 715                 720

Ile His Glu Pro Leu Pro Pro Pro Pro Pro Gln Arg Arg Asp Thr His
                725                 730                 735

Pro Ser Val Val Glu Thr Asp Gly His Ala Arg Val Val Val Pro Thr
            740                 745                 750

Leu Lys Gln His Ser His Pro Pro Pro Leu Ala Leu Gly Ser Gly Leu
        755                 760                 765

His Ala Pro His Lys Gly Pro Leu Pro Gln Ala Ser Asp Pro Ala Val
    770                 775                 780

Ala Arg Gln His Arg Pro Leu Pro Ser Thr Pro Asp Ser Ser His His
785                 790                 795                 800

Ala Gln Ala Thr Pro Arg Trp Arg Tyr Asn Lys Pro Leu Pro Pro Thr
                805                 810                 815

Pro Asp Leu Pro Gln Pro His Leu Pro Pro Ile Ser Ala Pro Gly Ser
            820                 825                 830

Ser Arg Ile Tyr Arg Pro Leu Pro Pro Leu Pro Ile Ile Asp Pro Pro
        835                 840                 845

Thr Glu Pro Pro Pro Leu Pro Pro Lys Ser Arg Gly Arg Ser Arg Ser
    850                 855                 860

Thr Arg Gly Gly His Met Asn Ser Gly Gly His Ala Lys Thr Arg Pro
865                 870                 875                 880

Ala Cys Gln Asp Trp Thr Val Pro Leu Pro Ala Ser Ala Gly Arg Thr
                885                 890                 895

Ser Trp Pro Pro Ala Thr Ala Arg Ser Thr Glu Ser Phe Thr Ser Thr
            900                 905                 910

Ser Arg Ser Lys Ser Glu Val Ser Pro Gly Met Ala Phe Ser Asn Met
        915                 920                 925

Thr Asn Phe Leu Cys Pro Ser Ser Pro Thr Thr Pro Trp Thr Pro Glu
    930                 935                 940

Leu Gln Gly Pro Thr Ser Lys Asp Glu Ala Gly Val Ser Glu His Pro
945                 950                 955                 960

Glu Ala Pro Ala Arg Glu Pro Leu Arg Arg Thr Thr Pro Gln Gln Gly
                965                 970                 975

Ala Ser Gly Pro Gly Arg Ser Pro Val Gly Gln Ala Arg Gln Pro Glu
            980                 985                 990

Lys Pro Ser His Leu His Leu Glu  Lys Ala Ser Ser Trp  Pro His Arg
        995                 1000                 1005

Arg Asp  Ser Gly Arg Pro Pro  Gly Asp Ser Ser Gly  Gln Ala Val
    1010                 1015                 1020

Ala Pro  Ser Glu Gly Ala Asn  Lys His Lys Gly Trp  Ser Arg Gln
    1025                 1030                 1035

Gly Leu  Arg Arg Pro Ser Ile  Leu Pro Glu Gly Ser  Ser Asp Ser
    1040                 1045                 1050

Arg Gly  Pro Ala Val Glu Lys  His Pro Gly Pro Ser  Asp Thr Val
    1055                 1060                 1065

Val Phe Arg Glu Lys Lys Pro Lys Glu Val Met Gly Gly Phe Ser
    1070                1075                1080

Arg Arg Cys Ser Lys Leu Ile Asn Ser Ser Gln Leu Leu Tyr Gln
    1085                1090                1095

Glu Tyr Ser Asp Val Val Leu Asn Lys Glu Ile Gln Ser Gln Gln
    1100                1105                1110

Arg Leu Glu Ser Leu Ser Glu Thr Pro Gly Pro Ser Ser Pro Arg
    1115                1120                1125

Gln Pro Arg Lys Ala Leu Val Ser Ser Glu Ser Tyr Leu Gln Arg
    1130                1135                1140

Leu Ser Met Ala Ser Ser Gly Ser Leu Trp Gln Glu Ile Pro Val
    1145                1150                1155

Val Arg Asn Ser Thr Val Leu Leu Ser Met Thr His Glu Asp Gln
    1160                1165                1170

Lys Leu Gln Glu Val Lys Phe Glu Leu Ile Val Ser Glu Ala Ser
    1175                1180                1185

Tyr Leu Arg Ser Leu Asn Ile Ala Val Asp His Phe Gln Leu Ser
    1190                1195                1200

Thr Ser Leu Arg Ala Thr Leu Ser Asn Gln Glu His Gln Trp Leu
    1205                1210                1215

Phe Ser Arg Leu Gln Asp Val Arg Asp Val Ser Ala Thr Phe Leu
    1220                1225                1230

Ser Asp Leu Glu Glu Asn Phe Glu Asn Asn Ile Phe Ser Phe Gln
    1235                1240                1245

Val Cys Asp Val Val Leu Asn His Ala Pro Asp Phe Arg Arg Val
    1250                1255                1260

Tyr Leu Pro Tyr Val Thr Asn Gln Thr Tyr Gln Glu Arg Thr Phe
    1265                1270                1275

Gln Ser Leu Met Asn Ser Asn Ser Asn Phe Arg Glu Val Leu Glu
    1280                1285                1290

Lys Leu Glu Ser Asp Pro Val Cys Gln Arg Leu Ser Leu Lys Ser
    1295                1300                1305

Phe Leu Ile Leu Pro Phe Gln Arg Ile Thr Arg Leu Lys Leu Leu
    1310                1315                1320

Leu Gln Asn Ile Leu Lys Arg Thr Gln Pro Gly Ser Ser Glu Glu
    1325                1330                1335

Ala Glu Ala Thr Lys Ala His His Ala Leu Glu Gln Leu Ile Arg
    1340                1345                1350

Asp Cys Asn Asn Asn Val Gln Ser Met Arg Arg Thr Glu Glu Leu
    1355                1360                1365

Ile Tyr Leu Ser Gln Lys Ile Glu Phe Glu Cys Lys Ile Phe Pro
    1370                1375                1380

Leu Ile Ser Gln Ser Arg Trp Leu Val Lys Ser Gly Glu Leu Thr
    1385                1390                1395

Ala Leu Glu Phe Ser Ala Ser Pro Gly Leu Arg Arg Lys Leu Asn
    1400                1405                1410

Thr Arg Pro Val His Leu His Leu Phe Asn Asp Cys Leu Leu Leu
    1415                1420                1425

Ser Arg Pro Arg Glu Gly Ser Arg Phe Leu Val Phe Asp His Ala
    1430                1435                1440

Pro Phe Ser Ser Ile Arg Gly Glu Lys Cys Glu Met Lys Leu His
    1445                1450                1455

Gly Pro His Lys Asn Leu Phe Arg Leu Phe Leu Arg Gln Asn Thr
    1460                1465                1470

Gln Gly Ala Gln Ala Glu Phe Leu Phe Arg Thr Glu Thr Gln Ser
    1475                1480                1485

Glu Lys Leu Arg Trp Ile Ser Ala Leu Ala Met Pro Arg Glu Glu
    1490                1495                1500

Leu Asp Leu Leu Glu Cys Tyr Asn Ser Pro Gln Val Gln Cys Leu
    1505                1510                1515

Arg Ala Tyr Lys Pro Arg Glu Asn Asp Glu Leu Ala Leu Glu Lys
    1520                1525                1530

Ala Asp Val Val Met Val Thr Gln Gln Ser Ser Asp Gly Trp Leu
    1535                1540                1545

Glu Gly Val Arg Leu Ser Asp Gly Glu Arg Gly Trp Phe Pro Val
    1550                1555                1560

Gln Gln Val Glu Phe Ile Ser Asn Pro Glu Val Arg Ala Gln Asn
    1565                1570                1575

Leu Lys Glu Ala His Arg Val Lys Thr Ala Lys Leu Gln Leu Val
    1580                1585                1590

Glu Gln Gln Ala
    1595

<210>  3
<211>  3519
<212>  DNA
<213>  homo sapiens

<220>

<221>  misc_feature
<223>  A partial sequence of SEQ ID NO:1, consisting of the nucleotides
       from the 175th to the 3693rd, which does not contain a region enc
       oding Dbl homology domain and Pleckstrin homology domain.


<220>
<221>  misc_feature
<223>  A polynucleotide encoding the amino acid sequence of SEQ ID NO:4.


<220>
<221>  CDS
<222>  (1)..(3519)
<223>

<400>  3
atg gag gct gag gag gcc cag cgt gga gcc tct cct ccc atc tct gcc        48
Met Glu Ala Glu Glu Ala Gln Arg Gly Ala Ser Pro Pro Ile Ser Ala
1               5                   10                  15

ata gag gaa ttc agc att atc cct gag gct ccc atg agg agc agc cag        96
Ile Glu Glu Phe Ser Ile Ile Pro Glu Ala Pro Met Arg Ser Ser Gln
            20                  25                  30

gtc tct gcc ttg ggg ctt gaa gcc caa gaa gat gag gac cca tcc tat       144
Val Ser Ala Leu Gly Leu Glu Ala Gln Glu Asp Glu Asp Pro Ser Tyr
        35                  40                  45

aag tgg aga gag gaa cac aga ctc tca gca act cag cag agt gag tta       192
Lys Trp Arg Glu Glu His Arg Leu Ser Ala Thr Gln Gln Ser Glu Leu
    50                  55                  60

agg gat gtg tgt gac tat gcg att gag acg atg ccc tct ttt ccc aag       240
Arg Asp Val Cys Asp Tyr Ala Ile Glu Thr Met Pro Ser Phe Pro Lys
65                  70                  75                  80

gaa ggt tct gca gat gtg gag ccc aat cag gaa agc ctt gtg gct gag       288
Glu Gly Ser Ala Asp Val Glu Pro Asn Gln Glu Ser Leu Val Ala Glu
                85                  90                  95

gcc tgt gac act ccg gaa cac tgg gag gca gta ccc cag agc cta gca       336
Ala Cys Asp Thr Pro Glu His Trp Glu Ala Val Pro Gln Ser Leu Ala
            100                 105                 110

ggc cga caa gca agg act cta gct ccc cca gag ctc tgg gcc tgc ccc       384
Gly Arg Gln Ala Arg Thr Leu Ala Pro Pro Glu Leu Trp Ala Cys Pro
        115                 120                 125

att cag agt gag cat cta gac atg gcc cca ttt tcc agt gac ctg gga       432
Ile Gln Ser Glu His Leu Asp Met Ala Pro Phe Ser Ser Asp Leu Gly
    130                 135                 140

agc gaa gaa gag gag gtg gaa ttt tgg cca gga ctt act tct ttg aca       480
Ser Glu Glu Glu Glu Val Glu Phe Trp Pro Gly Leu Thr Ser Leu Thr
145                 150                 155                 160

ttg gga tct gga cag gca gaa gaa gaa gag gaa acc tct tca gat aac       528
Leu Gly Ser Gly Gln Ala Glu Glu Glu Glu Glu Thr Ser Ser Asp Asn
                165                 170                 175

tct ggt cag acc aga tat tat tct ccc tgc gaa gag cat cct gca gag       576
Ser Gly Gln Thr Arg Tyr Tyr Ser Pro Cys Glu Glu His Pro Ala Glu
            180                 185                 190

acc aac cag aat gaa ggc tct gaa agt ggg act atc agg cag ggg gaa       624
Thr Asn Gln Asn Glu Gly Ser Glu Ser Gly Thr Ile Arg Gln Gly Glu
        195                 200                 205

gag ctg cca cct gag gag ctg cag gaa agt caa ggg ctc ttg cat ccc       672
Glu Leu Pro Pro Glu Glu Leu Gln Glu Ser Gln Gly Leu Leu His Pro
    210                 215                 220

cag gag gtc caa gtt ctg gag gag cag gga cag cag gaa gca gga ttt       720
Gln Glu Val Gln Val Leu Glu Glu Gln Gly Gln Gln Glu Ala Gly Phe
225                 230                 235                 240

cgg ggg gaa gga act ctg agg gag gat gtt tgt gcc gat ggg cta tta 768
Arg Gly Glu Gly Thr Leu Arg Glu Asp Val Cys Ala Asp Gly Leu Leu
                245                 250                 255

ggg gag gaa cag atg ata gag cag gtt aat gat gaa aag gga gaa cag 816
Gly Glu Glu Gln Met Ile Glu Gln Val Asn Asp Glu Lys Gly Glu Gln
            260                 265                 270

aag caa aaa cag gaa cag gta caa gat gtg atg ctt ggg aga caa gga 864
Lys Gln Lys Gln Glu Gln Val Gln Asp Val Met Leu Gly Arg Gln Gly
        275                 280                 285

gaa aga atg ggg ctc act ggg gag cca gag ggt ctg aat gac ggt gag 912
Glu Arg Met Gly Leu Thr Gly Glu Pro Glu Gly Leu Asn Asp Gly Glu
    290                 295                 300

tgg gag cag gag gat atg gag agg aag gct cag ggt cag gga ggt cca 960
Trp Glu Gln Glu Asp Met Glu Arg Lys Ala Gln Gly Gln Gly Gly Pro
305                 310                 315                 320

gaa cag gga gaa gag agg aag agg gag ctg cag gtg cca gaa gag aat 1008
Glu Gln Gly Glu Glu Arg Lys Arg Glu Leu Gln Val Pro Glu Glu Asn
                325                 330                 335

agg gcg gac tct cag gac gaa aag agt caa acc ttt ttg gga aaa tca 1056
Arg Ala Asp Ser Gln Asp Glu Lys Ser Gln Thr Phe Leu Gly Lys Ser
            340                 345                 350

gag gaa gta act gga aag caa gaa gat cat ggt ata aag gag aaa ggg 1104
Glu Glu Val Thr Gly Lys Gln Glu Asp His Gly Ile Lys Glu Lys Gly
        355                 360                 365

gtg cca gtc agc ggg cag gag gcg aaa gag cca gag agt tgg gat ggg 1152
Val Pro Val Ser Gly Gln Glu Ala Lys Glu Pro Glu Ser Trp Asp Gly
    370                 375                 380

ggc agg ctg ggg gca gtg gga aga gcg agg agc agg gaa gag gag aat 1200
Gly Arg Leu Gly Ala Val Gly Arg Ala Arg Ser Arg Glu Glu Glu Asn
385                 390                 395                 400

gag cat cat ggg cct tca atg ccc gct ctg ata gcc cct gag gac tct 1248
Glu His His Gly Pro Ser Met Pro Ala Leu Ile Ala Pro Glu Asp Ser
                405                 410                 415

cct cac tgt gac ctg ttt cca ggt gcc tca tat ctc atg act cag att 1296
Pro His Cys Asp Leu Phe Pro Gly Ala Ser Tyr Leu Met Thr Gln Ile
            420                 425                 430

ccc ggg act cag aca gag tcc agg gct gag gaa ctg tcc ccc gca gct 1344
Pro Gly Thr Gln Thr Glu Ser Arg Ala Glu Glu Leu Ser Pro Ala Ala
        435                 440                 445

ctg tct ccc tcg tta gag ccc atc agg tgc tct cac cag ccc att tct 1392
Leu Ser Pro Ser Leu Glu Pro Ile Arg Cys Ser His Gln Pro Ile Ser
    450                 455                 460

cta ctg ggc tcc ttt ttg act gag gag tca cct gac aag gaa ata gat 1440
Leu Leu Gly Ser Phe Leu Thr Glu Glu Ser Pro Asp Lys Glu Ile Asp
465                 470                 475                 480

caa aac agc cag caa gag gga tcc agg ctg agg aag gga aca gtg tcc 1488
Gln Asn Ser Gln Gln Glu Gly Ser Arg Leu Arg Lys Gly Thr Val Ser
                485                 490                 495

agc caa ggg act gag gtg gtc ttt gcc agt gca tct gtg act cct cca 1536
Ser Gln Gly Thr Glu Val Val Phe Ala Ser Ala Ser Val Thr Pro Pro
            500                 505                 510

agg aca cca gat tca gct cct ccc agt cct gct gaa gcc tac ccc atc 1584
Arg Thr Pro Asp Ser Ala Pro Pro Ser Pro Ala Glu Ala Tyr Pro Ile
        515                 520                 525

aca cct gcc tcg gta tct gcc agg ccc cca gtt gcc ttt ccc agg agg 1632
Thr Pro Ala Ser Val Ser Ala Arg Pro Pro Val Ala Phe Pro Arg Arg
    530                 535                 540

gaa acc tct tgt gct gca cgt gct cca gaa act gcc agt gcc cct ctc 1680
Glu Thr Ser Cys Ala Ala Arg Ala Pro Glu Thr Ala Ser Ala Pro Leu
545                 550                 555                 560

tca atg gat gac cca tct ccc tgt ggg act tct gag atg tgc cag gct 1728
Ser Met Asp Asp Pro Ser Pro Cys Gly Thr Ser Glu Met Cys Gln Ala
                565                 570                 575

gcc ctc tat ggc ttc ccc tcc acc ggg acc agc cct cca agg ccc cca 1776
Ala Leu Tyr Gly Phe Pro Ser Thr Gly Thr Ser Pro Pro Arg Pro Pro
            580                 585                 590

gcc aac tcc aca ggc acc gtc cag cac tta cgg agt gac tcc ttc cct 1824
Ala Asn Ser Thr Gly Thr Val Gln His Leu Arg Ser Asp Ser Phe Pro
        595                 600                 605

ggt tct cac agg aca gag cag act cca gac ctg gtg gga atg ttg ctt 1872
Gly Ser His Arg Thr Glu Gln Thr Pro Asp Leu Val Gly Met Leu Leu
    610                 615                 620

tcc tac tcc cac tca gag ctg ccc cag agg ccc ccc aaa cct gcc atc 1920
Ser Tyr Ser His Ser Glu Leu Pro Gln Arg Pro Pro Lys Pro Ala Ile
625                 630                 635                 640

tac agc tct gtg acc cca aga agg gac aga agg agt ggt agg gac tac 1968
Tyr Ser Ser Val Thr Pro Arg Arg Asp Arg Arg Ser Gly Arg Asp Tyr
                645                 650                 655

agc acc gtt tca gca tcc cct act gcc tta tcc acg ctg aag cag gac 2016
Ser Thr Val Ser Ala Ser Pro Thr Ala Leu Ser Thr Leu Lys Gln Asp
            660                 665                 670

tct caa gaa tcc atc tca aat cta gag aga ccc agc agt cct ccc agc 2064
Ser Gln Glu Ser Ile Ser Asn Leu Glu Arg Pro Ser Ser Pro Pro Ser
        675                 680                 685

atc cag ccc tgg gtc tcc cca cat aat cca gcc ttt gcc aca gag tct 2112
Ile Gln Pro Trp Val Ser Pro His Asn Pro Ala Phe Ala Thr Glu Ser
    690                 695                 700

ccc gcc tac ggt tct tcc cca tcc ttt gtc tcc atg gag gat gtg agg 2160
Pro Ala Tyr Gly Ser Ser Pro Ser Phe Val Ser Met Glu Asp Val Arg
705                 710                 715                 720

atc cac gaa cct ctg ccc cct cct ccc cca cag agg agg gac acc cat 2208
Ile His Glu Pro Leu Pro Pro Pro Pro Pro Gln Arg Arg Asp Thr His
                725                 730                 735

ccc tcc gtg gtg gag aca gat ggc cat gct cgt gta gtg gtt ccc acg 2256
Pro Ser Val Val Glu Thr Asp Gly His Ala Arg Val Val Val Pro Thr
            740                 745                 750

ctg aag cag cat agc cac cct cct cca ttg gcc cta ggt tca ggg ctg 2304
Leu Lys Gln His Ser His Pro Pro Pro Leu Ala Leu Gly Ser Gly Leu
        755                 760                 765

cat gcc ccc cat aaa ggc cca ctt ccc caa gcc tct gac ccc gct gtg 2352
His Ala Pro His Lys Gly Pro Leu Pro Gln Ala Ser Asp Pro Ala Val
    770                 775                 780

gcc agg cag cac cga cct ctg cca tct acc cca gac agc tcc cac cat 2400
Ala Arg Gln His Arg Pro Leu Pro Ser Thr Pro Asp Ser Ser His His
785                 790                 795                 800

gct cag gcc acc ccc agg tgg aga tac aac aag ccg cta ccc cct acc 2448
Ala Gln Ala Thr Pro Arg Trp Arg Tyr Asn Lys Pro Leu Pro Pro Thr
                805                 810                 815

cct gat ttg ccg cag ccc cac ctt cct ccc att tct gct cct ggt agc 2496
Pro Asp Leu Pro Gln Pro His Leu Pro Pro Ile Ser Ala Pro Gly Ser
            820                 825                 830

tca agg atc tac agg cct cta ccc cca cta ccc atc ata gac cct ccc 2544
Ser Arg Ile Tyr Arg Pro Leu Pro Pro Leu Pro Ile Ile Asp Pro Pro
        835                 840                 845

acc gaa cca ccc cca ttg ccc cca aag tcc agg ggg agg agc agg agc 2592
Thr Glu Pro Pro Pro Leu Pro Pro Lys Ser Arg Gly Arg Ser Arg Ser
    850                 855                 860

act cgg gga gga cat atg aac tca ggg ggt cat gcc aaa aca aga cct 2640
Thr Arg Gly Gly His Met Asn Ser Gly Gly His Ala Lys Thr Arg Pro
865                 870                 875                 880

gct tgt caa gac tgg aca gtc ccc ctc cct gcc tct gct gga cgc acc 2688
Ala Cys Gln Asp Trp Thr Val Pro Leu Pro Ala Ser Ala Gly Arg Thr
                885                 890                 895

tcc tgg ccc ccg gcc aca gct aga tca aca gag tct ttc act tcc acc 2736
Ser Trp Pro Pro Ala Thr Ala Arg Ser Thr Glu Ser Phe Thr Ser Thr
            900                 905                 910

agc agg agt aag agc gaa gtg tcc cct ggc atg gct ttc agc aac atg 2784
Ser Arg Ser Lys Ser Glu Val Ser Pro Gly Met Ala Phe Ser Asn Met
        915                 920                 925

aca aac ttc cta tgc ccc tct tcc cct acc act ccc tgg act ccg gag 2832
Thr Asn Phe Leu Cys Pro Ser Ser Pro Thr Thr Pro Trp Thr Pro Glu
    930                 935                 940

ctc cag gga ccc acc tct aag gat gaa gca ggg gtc tca gaa cac cct 2880
Leu Gln Gly Pro Thr Ser Lys Asp Glu Ala Gly Val Ser Glu His Pro
945                 950                 955                 960

gag gcc cct gcg aga gaa cct ttg aga agg aca acc cct cag caa gga 2928
Glu Ala Pro Ala Arg Glu Pro Leu Arg Arg Thr Thr Pro Gln Gln Gly
                965                 970                 975

gcc agt ggc cca ggg agg tca cct gtg ggc caa gca agg cag cca gaa 2976
Ala Ser Gly Pro Gly Arg Ser Pro Val Gly Gln Ala Arg Gln Pro Glu
            980                 985                 990

aaa ccc agc cat ctg cac ctg gag aag gcg tcc agc tgg ccc cac agg 3024
Lys Pro Ser His Leu His Leu Glu Lys Ala Ser Ser Trp Pro His Arg
        995                 1000                1005

cgg gac tca ggg agg cca cca ggg gac agc agt gga cag gct gtg 3069
Arg Asp Ser Gly Arg Pro Pro Gly Asp Ser Ser Gly Gln Ala Val
    1010                1015                1020

gct cct agt gag ggg gcc aac aag cac aag ggc tgg agc cgg cag 3114
Ala Pro Ser Glu Gly Ala Asn Lys His Lys Gly Trp Ser Arg Gln
    1025                1030                1035

ggc ctg cgc aga cct tcc atc ttg cct gag ggc tct tca gat tca 3159
Gly Leu Arg Arg Pro Ser Ile Leu Pro Glu Gly Ser Ser Asp Ser
    1040                1045                1050

aga ggt cca gcc gtg gag aaa cat ccg gga ccc tca gac act gtt 3204
Arg Gly Pro Ala Val Glu Lys His Pro Gly Pro Ser Asp Thr Val
    1055                1060                1065

gtt ttt cgg gag aaa aaa cca aag gag gtg atg gga ggc ttt tca 3249
Val Phe Arg Glu Lys Lys Pro Lys Glu Val Met Gly Gly Phe Ser
    1070                1075                1080

aga cgc tgc tcc aaa ctc atc aac tcc tcc cag ctg ctt tac cag 3294
Arg Arg Cys Ser Lys Leu Ile Asn Ser Ser Gln Leu Leu Tyr Gln
    1085                1090                1095

gag tat agt gat gtt gtc ctg aat aag gag atc cag agc cag cag 3339
Glu Tyr Ser Asp Val Val Leu Asn Lys Glu Ile Gln Ser Gln Gln
    1100                1105                1110

cgg ctg gag agc ctg tcc gag aca ccc ggg cct agc tct ccg cgg 3384
Arg Leu Glu Ser Leu Ser Glu Thr Pro Gly Pro Ser Ser Pro Arg
    1115                1120                1125

cag cct cgg aag gcc ctg gtc tcc tcc gag tcg tac ctg cag cgg 3429
Gln Pro Arg Lys Ala Leu Val Ser Ser Glu Ser Tyr Leu Gln Arg
    1130                1135                1140

ctc tcc atg gcc tcc agc ggc tcc ctc tgg cag gaa atc ccc gtg 3474
Leu Ser Met Ala Ser Ser Gly Ser Leu Trp Gln Glu Ile Pro Val
    1145                1150                1155

gtg cgc aac agc acc gtg ctg ctc tcc atg acc cat gaa gac caa 3519
Val Arg Asn Ser Thr Val Leu Leu Ser Met Thr His Glu Asp Gln
    1160                1165                1170

<210> 4
<211> 1173
<212> PRT
<213> homo sapiens

<400> 4

Met Glu Ala Glu Glu Ala Gln Arg Gly Ala Ser Pro Pro Ile Ser Ala
1               5                   10                  15

Ile Glu Glu Phe Ser Ile Ile Pro Glu Ala Pro Met Arg Ser Ser Gln
            20                  25                  30

Val Ser Ala Leu Gly Leu Glu Ala Gln Glu Asp Glu Asp Pro Ser Tyr
        35                  40                  45

Lys Trp Arg Glu Glu His Arg Leu Ser Ala Thr Gln Gln Ser Glu Leu
    50                  55                  60

Arg Asp Val Cys Asp Tyr Ala Ile Glu Thr Met Pro Ser Phe Pro Lys
65                  70                  75                  80

Glu Gly Ser Ala Asp Val Glu Pro Asn Gln Glu Ser Leu Val Ala Glu
                85                  90                  95

Ala Cys Asp Thr Pro Glu His Trp Glu Ala Val Pro Gln Ser Leu Ala
            100                 105                 110

Gly Arg Gln Ala Arg Thr Leu Ala Pro Pro Glu Leu Trp Ala Cys Pro
        115                 120                 125

Ile Gln Ser Glu His Leu Asp Met Ala Pro Phe Ser Ser Asp Leu Gly
    130                 135                 140

Ser Glu Glu Glu Glu Val Glu Phe Trp Pro Gly Leu Thr Ser Leu Thr
145                 150                 155                 160

Leu Gly Ser Gly Gln Ala Glu Glu Glu Glu Glu Thr Ser Ser Asp Asn
                165                 170                 175

Ser Gly Gln Thr Arg Tyr Tyr Ser Pro Cys Glu Glu His Pro Ala Glu
            180                 185                 190

Thr Asn Gln Asn Glu Gly Ser Glu Ser Gly Thr Ile Arg Gln Gly Glu
        195                 200                 205

Glu Leu Pro Pro Glu Glu Leu Gln Glu Ser Gln Gly Leu Leu His Pro
    210                 215                 220

Gln Glu Val Gln Val Leu Glu Glu Gln Gly Gln Gln Glu Ala Gly Phe
225                 230                 235                 240

Arg Gly Glu Gly Thr Leu Arg Glu Asp Val Cys Ala Asp Gly Leu Leu
                245                 250                 255

Gly Glu Glu Gln Met Ile Glu Gln Val Asn Asp Glu Lys Gly Glu Gln
            260                 265                 270

Lys Gln Lys Gln Glu Gln Val Gln Asp Val Met Leu Gly Arg Gln Gly
        275                 280                 285

Glu Arg Met Gly Leu Thr Gly Glu Pro Glu Gly Leu Asn Asp Gly Glu
    290                 295                 300

Trp Glu Gln Glu Asp Met Glu Arg Lys Ala Gln Gly Gln Gly Gly Pro
305                 310                 315                 320

Glu Gln Gly Glu Glu Arg Lys Arg Glu Leu Gln Val Pro Glu Glu Asn
                325                 330                 335

Arg Ala Asp Ser Gln Asp Glu Lys Ser Gln Thr Phe Leu Gly Lys Ser
            340                 345                 350

Glu Glu Val Thr Gly Lys Gln Glu Asp His Gly Ile Lys Glu Lys Gly
        355                 360                 365

Val Pro Val Ser Gly Gln Glu Ala Lys Glu Pro Glu Ser Trp Asp Gly
    370                 375                 380

Gly Arg Leu Gly Ala Val Gly Arg Ala Arg Ser Arg Glu Glu Glu Asn
385                 390                 395                 400

Glu His His Gly Pro Ser Met Pro Ala Leu Ile Ala Pro Glu Asp Ser
                405                 410                 415

Pro His Cys Asp Leu Phe Pro Gly Ala Ser Tyr Leu Met Thr Gln Ile
            420                 425                 430

Pro Gly Thr Gln Thr Glu Ser Arg Ala Glu Glu Leu Ser Pro Ala Ala
        435                 440                 445

Leu Ser Pro Ser Leu Glu Pro Ile Arg Cys Ser His Gln Pro Ile Ser
    450                 455                 460

Leu Leu Gly Ser Phe Leu Thr Glu Glu Ser Pro Asp Lys Glu Ile Asp
465                 470                 475                 480

Gln Asn Ser Gln Gln Glu Gly Ser Arg Leu Arg Lys Gly Thr Val Ser
                485                 490                 495

Ser Gln Gly Thr Glu Val Val Phe Ala Ser Ala Ser Val Thr Pro Pro
            500                 505                 510

Arg Thr Pro Asp Ser Ala Pro Pro Ser Pro Ala Glu Ala Tyr Pro Ile
        515                 520                 525

Thr Pro Ala Ser Val Ser Ala Arg Pro Pro Val Ala Phe Pro Arg Arg
    530                 535                 540

Glu Thr Ser Cys Ala Ala Arg Ala Pro Glu Thr Ala Ser Ala Pro Leu
545                 550                 555                 560

Ser Met Asp Asp Pro Ser Pro Cys Gly Thr Ser Glu Met Cys Gln Ala
                565                 570                 575

Ala Leu Tyr Gly Phe Pro Ser Thr Gly Thr Ser Pro Pro Arg Pro Pro
            580                 585                 590

Ala Asn Ser Thr Gly Thr Val Gln His Leu Arg Ser Asp Ser Phe Pro
        595                 600                 605

Gly Ser His Arg Thr Glu Gln Thr Pro Asp Leu Val Gly Met Leu Leu
    610                 615                 620

Ser Tyr Ser His Ser Glu Leu Pro Gln Arg Pro Pro Lys Pro Ala Ile
625                 630                 635                 640

Tyr Ser Ser Val Thr Pro Arg Arg Asp Arg Arg Ser Gly Arg Asp Tyr
                645                 650                 655

Ser Thr Val Ser Ala Ser Pro Thr Ala Leu Ser Thr Leu Lys Gln Asp
            660                 665                 670

Ser Gln Glu Ser Ile Ser Asn Leu Glu Arg Pro Ser Ser Pro Pro Ser
        675                 680                 685

Ile Gln Pro Trp Val Ser Pro His Asn Pro Ala Phe Ala Thr Glu Ser
    690                 695                 700

Pro Ala Tyr Gly Ser Ser Pro Ser Phe Val Ser Met Glu Asp Val Arg
705                 710                 715                 720

Ile His Glu Pro Leu Pro Pro Pro Pro Pro Gln Arg Arg Asp Thr His
                725                 730                 735

Pro Ser Val Val Glu Thr Asp Gly His Ala Arg Val Val Val Pro Thr
            740                 745                 750

Leu Lys Gln His Ser His Pro Pro Pro Leu Ala Leu Gly Ser Gly Leu
        755                 760                 765

His Ala Pro His Lys Gly Pro Leu Pro Gln Ala Ser Asp Pro Ala Val
    770                 775                 780

Ala Arg Gln His Arg Pro Leu Pro Ser Thr Pro Asp Ser Ser His His
785                 790                 795                 800

Ala Gln Ala Thr Pro Arg Trp Arg Tyr Asn Lys Pro Leu Pro Pro Thr
                805                 810                 815

Pro Asp Leu Pro Gln Pro His Leu Pro Pro Ile Ser Ala Pro Gly Ser
            820                 825                 830

Ser Arg Ile Tyr Arg Pro Leu Pro Pro Leu Pro Ile Ile Asp Pro Pro
        835                 840                 845

Thr Glu Pro Pro Pro Leu Pro Pro Lys Ser Arg Gly Arg Ser Arg Ser
    850                 855                 860

Thr Arg Gly Gly His Met Asn Ser Gly Gly His Ala Lys Thr Arg Pro
865                 870                 875                 880

Ala Cys Gln Asp Trp Thr Val Pro Leu Pro Ala Ser Ala Gly Arg Thr
                885                 890                 895

Ser Trp Pro Pro Ala Thr Ala Arg Ser Thr Glu Ser Phe Thr Ser Thr
            900                 905                 910

Ser Arg Ser Lys Ser Glu Val Ser Pro Gly Met Ala Phe Ser Asn Met
        915                 920                 925

Thr Asn Phe Leu Cys Pro Ser Ser Pro Thr Thr Pro Trp Thr Pro Glu
    930                 935                 940

Leu Gln Gly Pro Thr Ser Lys Asp Glu Ala Gly Val Ser Glu His Pro
945                 950                 955                 960

Glu Ala Pro Ala Arg Glu Pro Leu Arg Arg Thr Thr Pro Gln Gln Gly
                965                 970                 975

Ala Ser Gly Pro Gly Arg Ser Pro Val Gly Gln Ala Arg Gln Pro Glu
            980                 985                 990

Lys Pro Ser His Leu His Leu Glu  Lys Ala Ser Ser Trp  Pro His Arg
        995                 1000                 1005

Arg Asp  Ser Gly Arg Pro Pro  Gly Asp Ser Ser Gly  Gln Ala Val
    1010                 1015                 1020

Ala Pro  Ser Glu Gly Ala Asn  Lys His Lys Gly Trp  Ser Arg Gln
    1025                 1030                 1035

Gly Leu  Arg Arg Pro Ser Ile  Leu Pro Glu Gly Ser  Ser Asp Ser
    1040                 1045                 1050

Arg Gly  Pro Ala Val Glu Lys  His Pro Gly Pro Ser  Asp Thr Val
    1055                 1060                 1065

Val Phe  Arg Glu Lys Lys Pro  Lys Glu Val Met Gly  Gly Phe Ser
    1070                 1075                 1080

Arg Arg  Cys Ser Lys Leu Ile  Asn Ser Ser Gln Leu  Leu Tyr Gln
    1085                 1090                 1095

Glu Tyr  Ser Asp Val Val Leu  Asn Lys Glu Ile Gln  Ser Gln Gln
    1100                 1105                 1110

Arg Leu  Glu Ser Leu Ser Glu  Thr Pro Gly Pro Ser  Ser Pro Arg
    1115                 1120                 1125

Gln Pro  Arg Lys Ala Leu Val  Ser Ser Glu Ser Tyr  Leu Gln Arg
    1130                 1135                 1140

Leu Ser Met Ala Ser Ser Gly Ser Leu Trp Gln Glu Ile Pro Val
    1145                1150                1155

Val Arg Asn Ser Thr Val Leu Leu Ser Met Thr His Glu Asp Gln
    1160                1165                1170

<210> 5
<211> 25
<212> DNA
<213> Artificial

<220>
<223> Designed oligonucleotide based on the sequence of SEQ ID NO:1 for
      use as a primer.

<400> 5
caccatggag gctgaggagg cccag                                          25

<210> 6
<211> 24
<212> DNA
<213> Artificial

<220>
<223> Designed oligonucleotide based on the sequence of SEQ ID NO:1 for
      use as a primer.

<400> 6
ggcttgctgt tccaccagct gtag                                           24

<210> 7
<211> 25
<212> DNA
<213> Artificial

<220>
<223> Designed oligonucleotide based on the sequence of SEQ ID NO:1 for
      use as a primer.

<400> 7
ttggtcttca tgggtcatgg agagc                                          25

<210> 8
<211> 582
<212> DNA
<213> Homo Sapiens

<220>
<221> misc_feature
<223> A polynucleotide encoding RhoA.

<400> 8
atggctgcca tccggaagaa actggtgatt gttggtgatg gagcctgtgg aaagacatgc      60

ttgctcatag tcttcagcaa ggaccagttc ccagaggtgt atgtgcccac agtgtttgag     120

aactatgtgg cagatatcga ggtggatgga aagcaggtag agttggcttt gtgggacaca     180

gctgggcagg aagattatga tcgcctgagg cccctctcct acccagatac cgatgttata     240

ctgatgtgtt tttccatcga cagccctgat agtttagaaa acatcccaga aaagtggacc     300

ccagaagtca agcatttctg tcccaacgtg cccatcatcc tggttgggaa taagaaggat     360

cttcggaatg atgagcacac aaggcgggag ctagccaaga tgaagcagga gccggtgaaa     420

cctgaagaag gcagagatat ggcaaacagg attggcgctt ttgggtacat ggagtgttca     480

gcaaagacca aagatggagt gagagaggtt tttgaaatgg ctacgagagc tgctctgcaa     540

gctagacgtg ggaagaaaaa atctggttgc cttgtcttgt ga                    582

<210>  9
<211>  193
<212>  PRT
<213>  homo sapiens

<220>
<221>  misc_feature
<223>  RhoA

<400>  9

Met Ala Ala Ile Arg Lys Lys Leu Val Ile Val Gly Asp Gly Ala Cys
1               5                   10                  15

Gly Lys Thr Cys Leu Leu Ile Val Phe Ser Lys Asp Gln Phe Pro Glu
            20                  25                  30

Val Tyr Val Pro Thr Val Phe Glu Asn Tyr Val Ala Asp Ile Glu Val
        35                  40                  45

Asp Gly Lys Gln Val Glu Leu Ala Leu Trp Asp Thr Ala Gly Gln Glu
    50                  55                  60

Asp Tyr Asp Arg Leu Arg Pro Leu Ser Tyr Pro Asp Thr Asp Val Ile
65                  70                  75                  80

Leu Met Cys Phe Ser Ile Asp Ser Pro Asp Ser Leu Glu Asn Ile Pro
                85                  90                  95

Glu Lys Trp Thr Pro Glu Val Lys His Phe Cys Pro Asn Val Pro Ile
            100                 105                 110

Ile Leu Val Gly Asn Lys Lys Asp Leu Arg Asn Asp Glu His Thr Arg
        115                 120                 125

Arg Glu Leu Ala Lys Met Lys Gln Glu Pro Val Lys Pro Glu Glu Gly
    130                 135                 140

Arg Asp Met Ala Asn Arg Ile Gly Ala Phe Gly Tyr Met Glu Cys Ser
145                 150                 155                 160

Ala Lys Thr Lys Asp Gly Val Arg Glu Val Phe Glu Met Ala Thr Arg
                165                 170                 175

Ala Ala Leu Gln Ala Arg Arg Gly Lys Lys Lys Ser Gly Cys Leu Val
            180                 185                 190

Leu


<210>  10
<211>  29
<212>  DNA
<213>  Artificial

<220>
<223>  Designed oligonucleotide based on the sequence of SEQ ID NO:8 for
       use as a primer.

<400>  10
caccatggct gccatccgga agaaactgg                                          29


<210>  11
<211>  28
<212>  DNA
<213>  Artificial

<220>
<223>  Designed oligonucleotide based on the sequence of SEQ ID NO:8 for
        use as a primer.

<400>  11
   tcacaagaca aggcaaccag attttttc                                            28


<210>  12
<211>  44
<212>  DNA
<213>  Artificial

<220>
<223>  Designed oligonucleotide for use as a primer.

<400>  12
gactagttct agatcgcgag cggccgccct tttttttttt tttt                         44